# EUROPEAN PATENT SPECIFICATION

(11) **EP 1 513 928 B1**
(45) Date of publication and mention of the grant of the patent: **21.08.2019**
(21) Application number: 03736723.2
(22) Date of filing: 27.05.2003
(51) Int. Cl.: C12N 15/873, A61K 35/12, C12N 5/0735

(54) **A BANK OF STEM CELLS FOR PRODUCING CELLS FOR TRANSPLANTATION HAVING HLA ANTIGENS MATCHING THOSE OF TRANSPLANT RECIPIENTS, AND METHODS FOR MAKING AND USING SUCH A STEM CELL BANK**
STAMMZELLBANK ZUR PRODUKTION VON DEN HLA-ANTIGENEN VON TRANSPLANTATIONSEMPFÄNGERN ENTSPRECHENDE HLA-ANTIGENE AUFWEISENDEN ZELLEN FÜR DIE TRANSPLANTATION UND VERFAHREN ZUR HERSTELLUNG UND ZUR VERWENDUNG EINER SOLCHEN ST AMMZELLBANK
BANQUE DE CELLULES SOUCHES DESTINEES A LA PRODUCTION DE CELLULES POUR TRANSPLANTATION POSSEDANT DES ANTIGENES HLA CORRESPONDANT A CEUX DES RECEVEURS DE TRANSPLANT, ET PROCEDES DE CONSTITUTION ET D'UTILISATION D'UNE TELLE BANQUE DE CELLULES SOUCHES

(30) Priority: 24.05.2002 US 382616 P; 21.02.2003 US 448585 P
(43) Date of publication of application: 16.03.2005
(60) Divisional application: 11179101.8
(73) Proprietor: Advanced Cell Technology, Inc., Worcester, MA 01605 (US)
(72) Inventor: WEST, Michael, D., Worcester, MA 01605 (US)
(74) Representative: Daniels, Jeffrey Nicholas
(86) International application number: PCT/US2003/016626
(87) International publication number: WO 2003/100018

(56) References cited:
- WO-A-02/102997
- WO-A1-01/30978
- WO-A2-03/046141
- US-A- 5 004 681
- US-A- 5 192 553
- US-A- 5 993 387
- US-A1- 2002 155 601
- LANZA R P ET AL: "Generation of histocompatible tissues using nuclear transplantation" NATURE BIOTECHNOLOGY, NATURE PUBLISHING GROUP, NEW YORK, NY, US, vol. 20, July 2002 (2002-07), pages 689-696, XP002955120 ISSN: 1087-0156
- FADEN RUTH R ET AL: "PUBLIC STEM CELL BANKS: CONSIDERATIONS OF JUSTICE IN STEM CELL RESEARCH AND THERAPY" HASTINGS CENTER REPORT, INSTITUTE OF SOCIETY, ETHICS AND THE LIFE SCIENCES, HASTINGS-O, US, vol. 33, no. 6, November 2003 (2003-11), pages 13-27, XP009077152 ISSN: 0093-0334
- TAYLOR ET AL: "Banking on human embryonic stem cells: estimating the number of donor cell lines needed for HLA matching" LANCET THE, LANCET LIMITED. LONDON, GB, vol. 366, no. 9502, 10 December 2005 (2005-12-10), pages 2019-2025, XP005204962 ISSN: 0140-6736
- BRADLEY J.: 'Stem cell medicine encounters the immune system' NATURE REVIEWS - IMMUNOLOGY vol. 2, no. 11, November 2002, pages 859 - 871, XP002981993
- WOLF S.A.: 'Storing lifeblood. Cord blood stem cell banking' AM. J. OF NURSING vol. 99, no. 8, August 1999, pages 60 - 63, XP002981994
- VOGEL G.: 'BRITISH SCIENCE: Pioneering Stem Cell Bank Will Soon Be Open for Deposits' SCIENCE vol. 297, no. 5588, 13 September 2002, page 1784, XP002981915
- ADAMSON J.W.: 'Cord blood stem cell banking and transplantation' STEM CELLS vol. 15, no. SUPPL. 1, 1997, pages 57 - 61, XP002982306
- HARRIS D.T.: 'Experience in autologous and allogeneic cord blood banking' J. OF HEMATOTHERAPY vol. 5, no. 2, April 1996, pages 123 - 128, XP001018554
- KOEGLER G. ET AL: 'HLA typing strategies of cord blood (CB) for unrelated stem cell banking within eurocord' HUMAN IMMUNOLOGY vol. 47, no. 1-2, ABSTRACT #P474, 1996, page 87, XP002981916
- CIBELLI JOSE B ET AL: "Parthenogenetic Stem Cells in Nonhuman Primates", SCIENCE, AMERICAN ASSOCIATION FOR THE ADVANCEMENT OF SCIENCE, WASHINGTON, DC; US, vol. 295, no. 5556, 1 February 2002 (2002-02-01), page 819, XP008091029, ISSN: 0036-8075, DOI: 10.1126/SCIENCE.1065637
- KIM KITAI ET AL: "Histocompatible embryonic stem cells by parthenogenesis", SCIENCE, AMERICAN ASSOCIATION FOR THE ADVANCEMENT OF SCIENCE, WASHINGTON, DC; US, vol. 315, no. 5811, 26 January 2007 (2007-01-26), pages 482-486, XP002467552, ISSN: 0036-8075, DOI: 10.1126/SCIENCE.1133542
- CIBELLI JOSE B ET AL: "Embryonic stem cells from parthenotes", METHODS IN ENZYMOLOGY, ACADEMIC PRESS, US, vol. 418, 1 January 2006 (2006-01-01), pages 117-135, XP009095437, ISSN: 0076-6879, DOI: 10.1016/S0076-6879(06)18008-8
- JOSE B CIBELLI ET AL: "Somatic Cell Nuclear Transfer in Humans: Pronuclear and Early Embryonic Development", E-BIOMED, MARY-ANN LIEBERT, LARCHMONT, NY, US, vol. 2, no. 5, 26 November 2001 (2001-11-26), pages 25-31, XP002635246, ISSN: 1524-8909, DOI: 10.1089/152489001753262168
- MITALIPOV S M ET AL: "PARTHENOGENETIC ACTIVATION OF RHESUS MONKEY OOCYTES AND RECONSTRUCTED EMBRYOS", BIOLOGY OF REPRODUCTION, NEW YORK, NY [U.A.] : ACADEM. PRESS, US, vol. 65, no. 1, 1 July 2001 (2001-07-01), pages 253-259, XP001117868, ISSN: 0006-3363, DOI: 10.1095/BIOLREPROD65.1.253
- LIN H ET AL: "MULTILINEAGE POTENTIAL OF HOMOZYGOUS STEM CELLS DERIVED FROM METAPHASE II OOCYTES", STEM CELLS, ALPHAMED PRESS, DAYTON, OH, US, vol. 21, no. 2, 1 January 2003 (2003-01-01), pages 152-161, XP001157416, ISSN: 1066-5099, DOI: 10.1634/STEMCELLS.21-2-152

## Description

### FIELD OF THE INVENTION

The invention described herein relates to methods for producing a collection of human stem cell cultures each of which contains pluripotent stem cells that have genes encoding the same set of critical cell surface antigenic proteins, i.e. histocompatibility antigens(HLA antigens in the case of human) as are present on the cells of members of a human population. (By critical antigens is meant the set of antigens that form the major histocompatibility complex and other antigens such as blood group antigens that are involved in immuno-mediated rejection when collogenic cells and tissues are transplanted into donors that express a different set of histocompatibility and other critical antigens). The methods disclosed herein include deriving such human stem cell cultures from cells of early embryos produced by parthenogenesis. Described herein are methods wherein human and non-human stem cell cultures are induced to differentiate ex or in vivo into cell types that are useful for therapeutic cell transplantation; and to methods by which the differentiated cells are isolated from other cell types. The description also relates to methods in which stem cell-derived differentiated cells having a selected set of critical cell surface antigens are therapeutically transplanted or engrafted to a recipient, e.g., a human patient in need of a cell transplant having cells that express the same critical cell surface antigens. The description further relates to a collection or "bank" of cultures of different types of stem cells, each culture having a different set of genes encoding cell surface antigenic proteins present in a human population; to compositions comprising the individual stem cell cultures that make up such a stem cell bank; and to compositions comprising differentiated cells derived from such stem cells.

Preferably, stem cell banks produced according to the invention will comprise stem cell lines which are homozygous for MHC alleles which occur very frequently in the human population. Typically, a stem cell bank according to the invention will comprise at least 15 stem cell lines and more preferably at least 100 to 1000 stem cell lines. Thereby, the stem cell bank will provide maximal therapeutic and diagnostic efficacy as it will contain cells that are histocompatible for a wide range of potential transplant recipients.

### BACKGROUND OF THE INVENTION

### A. Histocompatibility and Transplant Rejection:

Histocompatibility is a largely unsolved problem in transplant medicine. Rejection of transplanted tissue is the result of an adaptive immune response to alloantigens on the grafted tissue by the transplant recipient. The alloantigens are "non-self' proteins, i.e., antigenic proteins that vary among individuals in the population and are identified as foreign by the immune system of a transplant recipient. The antigens on the surfaces of transplanted tissue that most strongly evoke rejection are the blood group (ABO) antigens and the major histocompatibity complex (MHC) proteins and in the case of humans, the human leukocyte antigen (HLA) proteins.

The blood group antigens were first described by Landsteiner in 1900; they are branched oligosaccharides that are attached to proteins and lipids on the surfaces of red blood cells, endothelial cells, and other cells, and are also present in secretions such as saliva. Compatibility of the blood group antigens of the ABO system of a vascularized organ or tissue transplant with those of the transplant recipient is generally required; but blood group compatibility may be unnecessary for many types of cell transplants.

The HLA proteins are encoded by clusters of genes that form a region located on chromosome 6 known as the Major Histocompatibility Complex, or MHC, in recognition of the important role of the proteins encoded by the MHC loci in graft rejection. Accordingly, the HLA proteins are also referred to as MHC proteins. The MHC genes and proteins will be used interchangeably in this application as the application encompasses human and non-human animal applications. The HLA or MHC proteins normally play a role in defending the body against foreign pathogens such as viruses, bacteria, and toxins. They are cell surface glycoproteins that bind peptides at intracellular locations and deliver them to the cell surface, where the combined ligand is recognized by a T cell. Class I MHC proteins are found on virtually all of the nucleated cells of the body. The class I MHC proteins bind peptides present in the cytosol and form peptide-MHC protein complexes that are presented at the cell surface, where they are recognized by cytotoxic CD8+ T cells. Class II MHC proteins are usually found only on antigen-presenting cells such as B lymphocytes, macrophages, and dendritic cells. The class II MHC proteins bind peptides present in a cell's vesicular system and form peptide-MHC protein complexes that are presented at the cell surface, where they are recognized by CD4+ T cells. CD4+ T cells activated by class II MHC proteins undergo clonal expansion with production of regulatory cytokines that signal helper and cytotoxic T cells. Unfortunately for those in need of transplants, the frequency of T cells in the body that are specific for non-self MHC molecules is relatively high, with the result that differences at MHC loci are the most potent critical elicitors of rejection of initial grafts. Rejection of most transplanted tissues is triggered predominantly by the recognition of class I MHC proteins as non-self proteins. T cell recognition of foreign antigens on the transplanted tissue sets in motion a chain of signaling and regulatory events that causes the activation and recruitment of additional T cells and other cytotoxic cells, and culminates in the destruction of the transplanted tissue. (Charles A. Janeway et al., Immunobiology, Garland Publishing, New York, NY, 2001, p. 524).

### B. The Genes Encoding MHC Proteins:

The MHC genes are polygenic - each individual possesses multiple, different MHC class I and MHC class II genes. The MHC genes are also polymorphic - many variants of each gene are present in the human and non-human population. In fact, the MHC genes are the most polymorphic genes known. Each MHC Class I receptor consists of a variable *α* chain and a relatively conserved *β*2-microglobulin chain. Three different, highly polymorphic class I *α* chain genes have been identified. These are called HLA-A, HLA-B, and HLA-C. Variations in the *α* chain chains account for all of the different class I MHC genes in the population. MHC Class II receptors are also made up of two polypeptide chains, an *α* chain and a *β* chain, both of which are polymorphic. In humans, there are three pairs of MHC class II *α* and *β* chain genes, called HLA-DR, HLA-DP, and HLA-DQ. Frequently, the HLA-DR cluster contains an extra gene encoding a *β* chain that can combine with the DR *α* chain; thus, an individual's three MHC Class II genes can give rise to four different MHC Class II molecules.

In humans, the genes encoding the MHC class I *α* chains and the MHC class II *α* and *β* chain are clustered on the short arm of chromosome 6 in a region that extends over from 4 to 7 million base pairs that is called the major histocompatibility complex. Every person usually inherits a copy of each HLA gene from each parent. If an individual's two alleles for a particular MHC locus encode structurally different proteins, the individual is heterozygous for that MHC allele. If an individual has two MHC alleles that encode the same MHC molecule, the individual is homozygous for that MHC allele. Because there are so many different variants of the MHC alleles in the population, most people have heterozygous MHC alleles. The numbers of different alleles found for each type of MHC class I *α* chain and MHC class II *α* and *β* chains as of January 2003 are shown in Table 1.

**TABLE 1. The numbers of different alleles for the polymorphic MHC class I and class II chains identified as of January, 2003.**

| MHC chain | no. of alleles |
|---|---|
| HLA-A | 266 |
| HLA-B | 511 |
| HLA-C | 6 |
| HLA-DRA | 3 |
| HLA-DRB | 403 |
| HLA-DQA1 | 23 |
| HLA-DQB1 | 53 |
| HLA-DPA1 | 20 |
| HLA-DPB1 | 101 |

The data in Table 1 is from the Internet web site of the Informatics Group of the Anthony Nolan Trust, The Royal Free Hospital, Hampstead, London, England. Lists of identified HLA Class I and Class II alleles are also available at the same web site.

### C. Matching MHC Types to Inhibit Rejection of Transplants:

Since the recognition that non-self MHC molecules are a major determinant of graft rejection, much effort has been put into developing assays to identify the MHC types present on the cells of tissue to be transplanted, and on the cells of transplant recipients, in order to match the types of MHC molecules present in the transplant tissue with those of the recipient. Tissue typing, the detection of MHC antigens, is performed by various means; for example, (i) by serology, using antibodies specific for particular MHC molecules to detect the presence of the targeted MHC molecules on donor or recipient cells, e.g., by the lymphocytotoxicity test; (ii) by detection of antibodies of a transplant recipient that bind specifically to a MHC protein of transplant tissue; and (iii) by direct analysis of the nucleotide sequence of the DNA of the MHC alleles. Most tissue typing for organ banking purposes is done by determining the blood type (ABO typing) and by typing the patient's and donor cells using serological methods; however, the use of rapid and reliable DNA-specific methods is increasing. Such methods can employ sequence-specific oligonucleotide primers and amplification by the polymerase chain reaction (PCR), and can be augmented by combining fluorescent detection methods with the use of a DNA chip to which are bound sequence specific oligonucleotides designed to detect unique sequences present in the different MHC alleles.

At present, tissue typing to match the HLA antigens of a transplant with those of a recipient is usually limited to the Class I HLA-A and -B antigens, and the Class II HLA-DR antigens. Most transplant donors are unrelated to the transplant recipient, and finding a tissue type to match that of the recipient usually involves matching the blood type and as many as possible of the 6 HLA alleles - two for each HLA-A, -B, and -DR locus. Transplant centers do not usually consider potential incompatibilites at other HLA loci, such as HLA-C and HLA-DPB1, although mismatches at these loci can also contribute to rejection. Considering only the combinations of maternal and paternal alleles of the HLA-A, HLA-B, and HLA-DR loci identified to date, there is a complexity of billions of possible tissue types. The task of matching HLA types of organs for transplant is simplified in that HLA-A and HLA-B are usually identified serologically. The number of HLA antigens identified serologically is considerably less than the number of different HLA antigens based on DNA sequencing. The World Health Organization (WHO) has recognized 28 distinct antigens in the HLA-A locus and 59 in the HLA-B locus, based on serological typing. Matching organs is also simplified to some extent by the fact that some alleles are much more common than others. Some of the more common HLA-A and HLA-B alleles are shown in Table 2:

**Table 2. Frequency of common HLA-A and HLA-B alleles in the population.**

| HLA-A (Frequency (%)) | | HLA-B (Frequency (%)) | |
|---|---|---|---|
| HLA-A1 | (25.1) | HLA-B5 | (15.2) |
| HLA-A2 | (44.8) | HLA-B7 | (18.2) |
| HLA-A3 | (22.6) | HLA-B8 | (16.7) |
| HLA-A24 | (18.2) | HLA-B12 | (32.5) |
| HLA-A11 | (11.8) | HLA-B14 | (8.8) |
| HLA-A28 | (9.8) | HLA-B18 | (11.3) |
| HLA-A29 | (10.3) | HLA-B35 | (15.2) |
| HLA-A32 | (9.8) | HLA-B40 | (13.7) |
| HLA-B15 | (12.3) | | |

(from Snell GD et al, Histocompatibility, New York, Academic Press, 1976)

The frequencies with which the various alleles appear in a population is not random; it depends on the racial makeup of the population. Dr. Motomi Mori has determined the frequencies with which thousand of different haplotypes of HLA-A, -B, and -DR loci appear in Caucasian, African-American, Asian-American, and Native American populations. Each haplotype is a particular combination of HLA-A, HLA-B, and HLA-DR loci that is present on a single copy of chromosome no. 6. The frequencies of several relatively common HLA-A, -B, and -DR haplotypes are shown in Table 3 to illustrate the wide variation in HLA haplotype frequencies in some of the racial groups that make up the North American population. In interpreting haplotype frequency data such as that shown in Table 3, one must bear in mind that cells of patients and organs are diploid and have an HLA type that is the product of the HLA haplotypes of the chromosomes inherited from both parents.

**Table 3. Examples of HLA-A, -B, -DR haplotype frequencies**

| HLA-A, -B, and -DR haplotype frequencies (expressed in percent) and their respective rankings within each racial group: Caucasian (CAU), African-American (AFR), Asian-American (ASI) and Native American (NAT). | | | | | | | | | | | | |
|---|---|---|---|---|---|---|---|---|---|---|---|---|
| Haplotype | | | | Frequency (%) | | | | Ranking | | | | |
| A | B | DR | CAU | AFR | ASI | LAT | NAT | CAU | AFR | ASI | LAT | NAT |
| 1 | 7 | 2 | 0.5349 | 0.2094 | 0.0798 | 0.1888 | 0.2812 | 21 | 58 | 262 | 91 | 62 |
| 1 | 8 | 3 | 5.1812 | 1.2491 | 0.3195 | 1.6733 | 4.7439 | 1 | 2 | 54 | 3 | 1 |
| 2 | 14 | 1 | 0.1563 | 0.0444 | 0.0076 | 0.3794 | 0.0624 | 107 | 539 | 1451 | 39 | 312 |
| 2 | 35 | 4 | 0.1457 | 0.0737 | 0.3293 | 1.2858 | 0.6342 | 115 | 302 | 49 | 4 | 12 |
| 2 | 35 | 8 | 0.0823 | 0.0931 | 0.1756 | 1.7641 | 0.3289 | 241 | 226 | 122 | 1 | 46 |
| 2 | 44 | 4 | 2.1507 | 0.6506 | 0.1276 | 0.6906 | 2.0004 | 3 | 4 | 170 | 12 | 3 |
| 3 | 7 | 2 | 2.6285 | 0.7596 | 0.1891 | 1.1986 | 2.7083 | 2 | 3 | 113 | 5 | 2 |
| 3 | 7 | 4 | 0.4411 | 0.1534 | 0.0498 | 0.1795 | 0.4448 | 30 | 104 | 408 | 98 | 29 |
| 3 | 7 | 8 | 0.0848 | 0.0367 | 0.0000 | 0.0622 | 0.0537 | 230 | 653 | 14053 | 310 | 366 |
| 3 | 35 | 1 | 1.0224 | 0.2741 | 0.1372 | 0.3552 | 0.8125 | 7 | 29 | 156 | 44 | 8 |
| 31 | 51 | 4 | 0.0915 | 0.0342 | 0.1646 | 0.2597 | 0.5691 | 209 | 699 | 135 | 64 | 16 |
| | 32 14 | 7 | 0.2617 | 0.0513 | 0.0046 | 0.1324 | 0.1775 | 57 | 479 | 1858 | 140 | 104 |

The data in Table 3 was produced for The National Marrow Donor Program Donor Registry, and is available at the Internet web site of Motomi Mori, Ph.D., Huntsman Cancer Institute, Salt Lake City, Utah.

### D. Rejection Triggered by Minor Histocompatibility Antigens:

Matching the MHC molecules of a transplant to those of the recipient significantly improves the success rate of clinical transplantation; however, it does not prevent rejection, even when the transplant is between HLA-identical siblings. This is because rejection is also triggered by differences between the minor histocompatibility antigens. These polymorphic antigens are actually "non-self" peptides bound to MHC molecules on the cells of the transplant tissue. The rejection response evoked by a single minor histocompatibility antigen is much weaker than that evoked by differences in MHC antigens, because the frequency of the responding T cells is much lower (Janeway et al., supra, page 525). Nonetheless, differences between minor histocompatibility antigens will often cause the immune system of a transplant recipient to eventually reject a transplant, even where there is a match between the MHC antigens, unless immunosuppressive drugs are used.

### E. Inadequate Supply of Cells, Tissues, and Organs for Transplant.

The number of people in need of cell, tissue, and organ transplants is far greater than the available supply of cells, tissues, and organs suitable for transplantation. Under these circumstances, it is not surprising that obtaining a good match between the MHC proteins of a recipient and those of the transplant is frequently impossible, and many transplant recipients must wait for an MHC-matched transplant to become available, or accept a transplant that is not MHC-matched. If the latter is necessary, the transplant recipient must rely on heavier doses of immunosuppressive drugs and face a greater risk of rejection than would be the case if MHC matching had been possible. There is presently a great need for new sources of cells, tissues, and organs suitable for transplantation that are histocompatible with the patients in need of such transplants.

WO 02/102997 A2 discloses isolated homozygous stem cells, differentiated cells derived therefrom and materials and methods for making and using same.

US 2002/155601 A1 discloses method for producing a population of homozygous stem cells having a pre-selected immunotype and/or genotype, cells suitable for transplant derived therefrom, and materials and methods using same.

Cibelli et al., discloses parthenogenetic stem cells in nonhuman primates (Science, 2002, vol. 295, no. 5556, page 819).

WO 01/30978 A1 discloses describes gynogenetic or androgenetic production of pluripotent cells and cell lines, and use thereof to produce differentiated cells and tissues.

WO 03/046141 A2, which is prior art under Article 54(3) EPC, discloses methods for making and using reprogrammed human somatic cell nuclei and autologous and isogenic human stem cells.

Cibelli et al., discloses somatic cell nuclear transfer in humans: pronuclear and early embryonic development (E-Biomed, 2001, vol. 2, no. 5, pages 25-31).

Lin et al., discloses multilineage potential of homozygous stem cells derived from metaphase II oocytes (Stem Cells, 2003, vol. 21, no. 2, pages 152-161).

The present invention provides a stem cell bank comprising a library or plurality of human parthenogenic stem cell lines, each of which is homozygous for at least one MHC allele present in a human population, wherein each member of said plurality of stem cell lines is homozygous for a different set of MHC alleles relative to the remaining members of the plurality of stem cell lines.

The present invention further provides an *in vitro* method for preparing a cell or tissue for transplant, comprising preparing cells or tissue for transplantation that are homozygous for at least one HLA allele in a person in need of such a transplant, comprising:
a. identifying the MHC alleles of a person in need of a transplant (the recipient);
b. obtaining stem cells which are homozygous for the MHC alleles identified in (a) from a stem cell bank comprising a plurality of human parthenogenic stem cells homozygous for at least one MHC allele of the transplant recipient; and
c. generating cells or tissue suitable for transplant from said stem cells.

IN addition the present invention provides an in vitro method for producing human stem cells homozygous for at least one MHC allele present in a human population, comprising:
a) activating oocytes;
b) exposing the activated oocytes to chemical treatment that inhibits extrusion of the second polar body;
c) culturing the embryo in vitro under conditions resulting in formation of a blastocyst;
d) culturing inner cell mass cells of the blastocyst in vitro under conditions resulting in production of stem cells; and
e) screening to identify stem cells homozygous in a particular MHC allele.

### BRIEF DESCRIPTION OF THE FIGURES

Figure 1 shows cynomulgous monkey blastocysts derived from parthenogenetic embryos.
Figure 2 shows an ES-like cell line (Cyno 1) derived from a cynomulgous parthenogenetic blastocyst.
Figure 3 and 4 show the Cyno 1 cell line before and after immunosurgery.
Figure 5 shows the Cyno 1 cell line 5 days after plating.
Figure 6 shows the Cyno 1 cell line growing on top of a feeder layer.
Figure 7 shows the results of an RT-PCR showing that the Cyno-1 cell line is homozygous for the Snrpn gene (contains paternal allele).
Figure 8 shows metaphase II oocytes at retrieval.
Figure 9 shows 4 and 6 cell embryo 48 hours after parthenogenetic activation.
Figure 10 shows blastocoele cavities in human parthenogenetic activation 48 hours after activation.
Figure 11 shows human parthenogenetic embryo having an inner cell mass.
Figure 12 shows human ES-like cells derived from cultured ICM cells.

### DESCRIPTION OF THE INVENTION

### A. A Bank Of Stem Cell Lines Homozygous For MHC Loci:

It is an object of the present invention to prepare a bank of parthenogenetically derived pluripotent stem cell lines that are homozygous for one or more MHC alleles that are present in the human population. Preferably, this work will be homozygous for MHC alleles that are representative of at least most prevalent in humans. Many of these lines will also have an ABO blood group type O-negative to make them broadly compatible across the different blood types. Stem cell lines of the stem cell bank of the present invention can be induced to differentiate into cell types suitable for therapeutic transplant. Because the cells produced by the methods of the present invention have
homozygous MHC alleles, the chance of obtaining cells for transplant that have MHC alleles that match those of a patient in need of a transplant is significantly enhanced. Instead of having to find a six of six match between two sets of HLA-A, HLA-B, and HLA-DR antigens, a high level of histocompatibility is provided by the cells for transplant
when either of the two HLA-A, HLA-B, and HLA-DR antigens of the prospective transplant recipient matches one of the corresponding homozygous HLA antigens of the cells for transplant. For example, a stem cell bank able to provide cells having an HLA-A/HLA-B match to a patient having any of the eight HLA-A and nine HLA-B antigens listed in Table 2 would require only 72 stem cell lines with homozygous HLA-A and HLA-B antigens; whereas a bank of stem cells with heterozygous HLA-A and HLA-B antigens would need to have 4032 different stem cell lines. To provide a library of heterozygous stem cell lines that match the WHO list of serological types would require obtaining stem cells having every combination of 28 different pairs of HLA-A antigens and 59 different pairs of HLA-B, to account for both the maternal and paternal alleles for each loci. The complexity of such a stem cell bank, i.e., the number of different cell lines required, would be 2,587,032. In contrast, a bank of stem cells homozygous for the same HLA-A and HLA-B antigens would only need to have a complexity of 1,652 stem cell lines to guarantee a match to a patient with HLA-A and -B antigens on the WHO list of serological types. The actual number required to meet the needs of a majority of patients will actually be less than this due to the non-random distribution of alleles in various populations around the world. Patients in need of bone marrow stem cell grafts who are homozygous in particular alleles are particularly sensitive to graft versus host disease when heterozygous bone marrow grafts are used. Stem cell grafts using stem cells having homozygous alleles made according to the methods of the present invention would alleviate this common complication of transplants.

This present invention provides novel means for making libraries of pluripotent stem cells that can serve as sources of cells for therapeutic transplant that are highly histocompatible with human or non-human patients in need of cell transplants. Additionally, those cell lines are useful in creating animal models for specific diseases that may be used to evaluate potential treatments and drug antidotes. In one embodiment, the invention comprises preparing a bank of stem cell lines that are homozygous for one or more critical antigen alleles, in the case of human stem cells. MHC alleles that are present in all or most of the world's populations, including the populations of North America, Central and South America, Europe, Africa, and Asia, and the Pacific islands.

An object of the present invention is to provide a stem cell bank comprising
human stem cells generated by parthenogenesis that are homozygous for MHC alleles.

The stem cell bank of the present invention may comprise lines of human parthenogenic pluripotent stem cells that are homozygous for at least one MHC allele selected from the group consisting of HLA-A, HLA-B, HLA-C, HLA-DR, HLA-DQ, and HLA-DP. In a useful embodiment, the stem cell bank comprises
pluripotent stem cells stem cells that are homozygous for the significant histocompatibility antigen alleles, e.g., the HLA-A, HLA-B, and HLA-DR alleles. In another embodiment, the stem cell bank comprises stem cells that are homozygous for all of the histocompatibility antigen alleles, i.e. MHC alleles.

The stem cell bank of the present invention can comprise parthenogenetically derived embryonic stem (ES) cells, that can differentiate in vivo or ex vivo into a wide variety of different cell types having one or more homozygous MHC alleles. The stem cell bank of the present invention can also comprise partially differentiated stem cells such as neuronal stem cells and/or hematopoietic stem cells, that differentiate in vivo or ex vivo into a more limited number of differentiated cell types having one or more homozygous MHC alleles. These stem cells optionally may be transgenic, e.g., they may express antigens that encode therapeutic or diagnostic proteins and polypeptides. For example, the stem cells may be genetically engineered to express proteins that inhibit immune rejection responses such as CD4O-L (CD154 or gp139).

An object of the present invention is to provide a stem cell bank comprising stem cells having homozygous histocompatibility alleles, i.e., MHC alleles that are available "off the shelf" for providing histocompatible cells suitable for transplant to patients in need of such a transplant. Desirably, this stem bank will include stem cell lines that are representative of the different histocompatibility antigens expressed in humans.
In a useful embodiment, the stem cell bank comprises stem cells that are isolated and maintained without feeder cells or serum of non-human animals, to minimize concerns of contamination by pathogens. In another useful embodiment, the stem cell bank comprises stem cells that are genetically modified relative to the cells of the donor, e.g., human donor from which they are derived.

Another object of the present description is to provide a stem cell bank comprising stem cells having homozygous recessive alleles responsible for genetically inherited diseases. Recessive disease-causing genes are endemic in the population, and such stem cells can be generated by parthenogenesis using oocytes collected from female carriers of the recessive disease-causing alleles. There is great need for
pluripotent stem cells that having homozygous recessive disease-causing alleles that can be induced to differentiate into cells useful for basic research directed to studying the disease phenotype, both ex vivo and in vivo (e.g., in immunodeficient laboratory animals), and for screening to discover drugs and other therapies that treat or cure the disease.

### B. Terms Used Herein:

As used herein, a "stem cell" is a cell that has the ability to proliferate in culture, producing some daughter cells that remain relatively undifferentiated, and other daughter cells that give rise to cells of one or more specialized cell types; and "differentiation" refers to a progressive, transforming process whereby a cell acquires the biochemical and morphological properties necessary to perform its specialized functions. Stem cells therefore reside immediately antecedent to the branch points of the developmental tree.

As used herein, an "embryonic stem cell line" is a cell line with the characteristics of the murine ES cells isolated from morulae or blastocyst inner cell masses, as reported by Martin (Proc. Natl. Acad. Sci. USA (1981) 78:7634-7638); and by Evans et al. (Nature (1981) 292: 154-156). ES cells have high nuclear-to-cytoplasm ratio, prominent nucleoli, are capable of proliferating indefinitely and can be differentiate into most or all of the specialized cell types of an organism, such as the three embryonic germ layers, all somatic cell lineages, and the germ line.

In some cases, ES cells are obtained that can differentiate into almost all of the specialized cell types of an organism; but not into one or a small number of specific cell types. For example, Thomson et al. describe isolating a primate ES cell that, when transferred into another blastocyst, does not contribute to the germ line (Proc. Natl. Acad. Sci. USA. (1995) 92:7844-7848).

According to claims 1 and 35 the stem cell bank comprises a plurality of human parthenogenetic stem cell lines.

As used herein, "inner cell mass-derived cells" (ICM-derived cells) are cells directly derived from isolated ICMs or morulae without passaging them to establish a continuous ES or ES-like cell line. Methods for making and using ICM-derived cells are described in co-owned U.S. Patent No. 6,235,970. According to the method of claim 57, the inner cell mass cells are of a blastocyst obtained by a method comprising: (a) activating oocytes; (b) exposing the activated oocytes to chemical treatment that inhibits extrusion of the second polar body; and (c) culturing the embryo *in vitro* under conditions resulting in formation of a blastocyst.

As used herein, "ex vivo" cell culture refers to culturing cells outside of the body. Ex vivo cell culture includes cell culture in vitro, e.g., in suspension, or in single- or multi-well plates. Ex vivo culture also includes co-culturing cells with two or more different cell types, and culturing in or on 2- or 3-dimensional supports or matrices, including methods for culturing cells alone or with other cell types to form artificial tissues.

As used herein, "parthenogenetic embryos" refers to an embryo that only contains male or female chromosomal DNA that is derived from male or female gametes. For example, parthenogenetic embryos can be derived by activation of unfertilized female gametes, e.g., unfertilized human, murine, cynomolgus or rabbit oocytes.

As used herein, the term "gene" refers to the nucleotide sequences at a genetic locus that encode and regulate expression of a functional mRNA molecule or a polypeptide; i.e., as used herein, a gene includes the nucleotide sequences that make up the coding sequence (exons and introns), the promoter, enhancers, and other DNA elements that regulate transcription, including as elements conferring cell type-specific and differentiation stage-specific expression, hormone responsive elements, repressor elements, etc., and nucleotide sequences that encode signals that regulate splicing and translation of the mRNA, such as a cleavage signal, a polyadenylation signal, or an internal ribosome entry site (IRES).

### C. Providing Histocompatible Transplants To Animal or Human Recipients:

Another object of the disclosure is to provide a method by which a human e.g., a person in need of a cell or tissue transplant can be provided with cells or tissue suitable for transplantation that have homozygous histocompatibility antigen alletes, e.g., in the case of human recipients MHC alleles that match the MHC alleles of the person needing the transplant. The invention provides a method in which the MHC alleles of a person in need of a transplant (the recipient) are identified, and a line of stem cells homozygous for at least one MHC allele present in the recipient's cells is obtained from a stem cell bank produced according to the disclosed methods. That line of stem cells is then used to generate cells or tissue suitable for transplant that are homozygous for at least one MHC allele present in the recipient's cells. The cells or tissues so obtained can be grafted to the body of the person in need of such a transplant. In a useful embodiment of the invention, three, four, five, six or more of the MHC alleles of the line of stem cells used to generate cells or tissue for transplant are homozygous and match MHC alleles of the transplant recipient.

In a useful embodiment, the line of stem cells used to generate cells or tissue suitable for transplant is a line of parthenogenetically derived embryonic stem cells.

The lines of stem cells that can be used to generate cells or tissue suitable for transplant are available "off the shelf" in the stem cell bank of the present invention.

In another useful embodiment, the stem cell bank of the present invention comprises one or more lines of pluripotent stem cell having DNA that is genetically modified relative to the DNA of the human donor from which they are derived. For example, the invention comprises altering genomic DNA of the cells to replace a non-homozygous MHC allele with one that is homozygous, or to inhibit the effective presentation of a class I or class II HLA antigen on the cell surface, e.g., by preventing expression of *β*2-microglobulin, or by preventing expression of one or more MHC alleles. Also, the invention encompasses introducing one or more genetic modifications that result in lineage-defective stem cells, i.e., stem cells which cannot differentiate into specific cell lineages.

### D. Methods For Making Stem Cell Lines With Homozygous MHC Alleles:

Pluripotent stem cell lines that make up the stem cell banks of the present invention can be derived from blastocyst embryos made up of cells that are homozygous for some or all of the histocompatibility antigen alleles, i.e. MHC alleles. Blastocyst embryos useful for the present invention are made by parthenogenesis.

In the case of human embryos, for ethical reasons, they are never allowed to develop beyond the stage of pre-implantation blastocysts of about 9-10 days before the inner cell mass cells are isolated and are cultured to produce embryonic stem (ES) cells.

### Stem cells produced by parthenogenesis:

According to the invention pluripotent human stem cells are derived from embryos produced by parthenogenesis. The stem cells obtained by this method are diploid, because extrusion of the second polar body following parthenogenetic activation is inhibited. Methods for producing a diploid human embryo by parthenogenesis, for culturing the embryo in vitro to form a blastocyst, and for culturing cells of the blastocyst to obtain stem cells, are described in co-owned and co-pending PCT Application PCT/USO2/37899 (Methods for Making and Using Reprogrammed Human Somatic Cell Nuclei and Autologous and Isogenic Stem Cells) filed November 26, 2002, and published as WO 03/046141 A2.

Similar methods for producing diploid embryos by parthenogenesis using oocytes of rhesus monkeys and cynomolgous monkeys have been described by Mitalipov et al. (2001, Biology of Reproduction, 65:253-259) and Cibelli et al. (2002, Science, 295:81), respectively.

In general, production of a diploid human embryo by parthenogenesis comprises
a. obtaining oocytes from human donors induced to superovulate by treatment with gonadotropins followed by hCG injection;
b. activating the oocytes at about 38-45 hours after hCG stimulation;
c. exposing the activated oocytes to chemical treatment that inhibits extrusion of the second polar body; and
d. culturing the embryo in vitro under conditions resulting in formation of a blastocyst.
Oocyte activation is normally mediated by oscillations of intracellular Ca⁺² ion triggered by the sperm cell. Parthenogenetic activation of the oocytes can be achieved by any of the known means for inducing oocyte activation. Such methods generally involve exposing the oocyte to ethanol, electroporation, calcium ionophore, ionomycin, or inositol 1,4,5-triphosphate to increase the intracellular Ca⁺² ion concentration in the oocyte, in combination with a treatment that temporarily inhibits protein synthesis or protein phosphorylation. For example, Mitalipov et al. (supra, p. 254) describe two such methods that result in production of diploid parthenogenetic blastocysts from oocytes of rhesus monkeys. In one method, the oocytes are incubated briefly in medium containing ionomycin and calcium, followed by incubation for several hours in medium containing 6-aminomethylpurine (DMAP), an inhibitor of protein phosphorylation. In the other method, the oocytes are electroporated three times in medium containing calcium, and between each electroporation, the oocytes are incubated for about 30 minutes in medium containing cycloheximide, an inhibitor of protein synthesis, and cytochalasin B, an inhibitor of microfilament synthesis.

Using a similar method Cibelli et al. (supra) parthenogenetically activated oocytes of a cynomolgous monkey; cultured the activated oocytes in vitro to produce a diploid blastocysts; and isolated a line of diploid ES cells from cells of the inner cell mass of a parthenogenesis-derived embryo; and showed that the ES cells are capable of differentiating into cell types of all three embryonic germ layers.

Oocytes are obtained from women having MHC alleles of the type needed for the stem cell bank. The oocytes are parthenogenetically activated and are cultured to form blastocysts. Using known methods, the inner cell mass cells of the blastocysts are cultured in vitro to generate diploid embryonic stem cells. Because extrusion of the second polar body after meiosis II was prevented, the homologous chromosomes of such ES cells are actually the sister chromatids that were joined together as a dyad during meiosis I. Since the sister chromatids were formed by replication of a single set of chromosomes at the outset of meiosis, they will have identical DNA sequences, except for those regions that were exchanged with the homologous dyad during the recombination stage of meiosis. The HLA genes of the MHC are tightly linked, and recombination in this region is rare. occurring with a frequency of about 1%. The two sets of homozygous HLA alleles in the parthenogenetically-derived stem cell lines obtained with oocytes from a given donor reflect the HLA haplotypes of the maternal and paternal copies of chromosome 6 that the donor inherited from her parents. Known screening methods can be performed to identify the cell lines that have non-homozygous HLA antigens due to genetic recombination, and to identify the homozygous HLA alleles of each stem cell line.

### Genetically modified stem cells:

The methods of the present invention include producing
pluripotent stem cells homozygous for MHC antigens that are genetically modified relative to the cells of the human donor from which they were originally obtained. The stem cells can be genetically modified in any manner that enhances or improves the overall efficiency by which cells for transplant are produced and the therapeutic efficacy of the cell transplantation. Methods that use recombinant DNA techniques to introduce modifications at selected sites in the genomic DNA of cultured cells are well known. Such methods can include (1) inserting a DNA sequence from another organism (human or non-human) into target nuclear DNA, (2) deleting one or more DNA sequences from target nuclear DNA, and (3) introducing one or more base mutations (e.g., site-directed mutations) into target nuclear DNA. Such methods are described, for example, in Molecular Cloning, a Laboratory Manual, 2nd Ed., 1989, Sambrook, Fritsch, and Maniatis, Cold Spring Harbor Laboratory Press; U.S. Pat. No. 5,633,067, "Method of Producing a Transgenic Bovine or Transgenic Bovine Embryo," DeBoer et al., issued May 27, 1997; U.S. Pat. No. 5,612,205, "Homologous Recombination in Mammalian Cells," Kay et al., issued Mar. 18, 1997; and PCT publication WO 93/22432, "Method for Identifying Transgenic Pre-Implantation Embryos ".

Such methods include techniques for transfecting cells with foreign DNA fragments and the proper design of the foreign DNA fragments such that they effect insertion, deletion, and/or mutation of the target DNA genome. For example, known methods for genetically altering cells that use homologous recombination can be used to insert, delete, or rearrange DNA sequences in the genome of a cell of the present invention. A genetic system that uses homologous recombination to modify targeted DNA sequences in a mammalian cell to "knock-out" a cell's ability to express a selected gene is disclosed by Capecchi et al. in U.S. Patent Nos. 5,631,153 and 5,464,764.

Such known methods can be used to insert into the genomic DNA of a cell an additional (exogenous) DNA sequence comprising an expression construct containing a gene that is to be expressed in the modified cell. The gene to be expressed can be operably linked to any of a wide variety of different types of transcriptional regulatory sequences that regulate expression of the gene in the modified cell. For example, the gene can be under control of a promoter that is constitutively active in many different cell types, or one that is developmentally regulated and is only active in one or a few specific cell types. Alternatively, the gene can be operably linked to an inducible promoter that can be activated by exposure of the cell to a physical (e.g., cold, heat, light, radiation) or chemical signal. Many such inducible promoters and methods for using them effectively are well known. Examples of the characteristics and use of such promoters, and of other well-known transcriptional regulatory elements such as enhancers, insulators, and repressors, are described, for example, in Transgenic Animals, Generation and Use, 1997, edited by L. M. Houdebine, Hardwood Academic Publishers, Australia.

Stem cells homozygous for MHC antigens that have multiple genetic alterations can be produced using known methods. For example, one can produce cells that are modified at multiple loci, or cells that are modified at a single locus by complex genetic alterations requiring multiple manipulations.

The pluripotent stem cells having homozygous MHC alleles that are produced by any of the methods described above can be genetically modified directly using known methods. For example, Zwaka et al. have described a method for genetically modifying human ES cells in vitro by homologous recombination (Nature Biotechnology, Vol. 21, No. 3, March, 2003).

Stem cells can be produced that are genetically modified grow more efficiently in tissue culture than unmodified cells; e.g., by increasing the number of growth factor receptors on the cells' surface. Use of stem cells having such modifications reduces the time required to generate an amount of cells for transplant that is sufficient to have therapeutic effect.

The histocompatibility of a line of cells to be used for transplant with a patient in need of such as transplant may be increased by altering the genomic DNA of the cells to replace a non-homozygous MHC allele with one that is homozygous and matches an HLA allele of the patient. Alternatively, the genomic DNA of the cells can be modified to inhibit the effective presentation of a class I or class II HLA antigen on the cell's surface; for example, by introducing a genetic alteration that prevents expression of *β*2-microglobulin, which is an essential component of class I HLA antigens; by introducing genetic alterations in the promoter regions of the class I and/or or class II MHC genes; or simply by deleting a portion of the DNA of one or more of the class I and/or or class II MHC genes sufficient to prevent expression of the gene(s).

Stem cells can be genetically modified (e.g., by homologous recombination) to have a heterozygous knock-out of the Id1 gene, and a homozygous knockout of the Id3 gene. As described in co-owned and co-pending PCT Application No. PCT/USU3/01827 (Stem Cell-Derived Endothelial Cells Modified to Disrupt Tumor Angiogenesis), filed January 22, 2003, and published as WO 03/061591 A2, these stem cells can be induced to differentiate into Id1+/-, Id3 -/- endothelial cell precursor cells that are useful for the treatment of
cancer because they give rise to endothelial cells that disrupt and inhibit tumor angiogenesis.

Stem cells can also be genetically modified to provide a therapeutic gene product that the patient requires, e.g., due to an inborn error of metabolism. Many genetic diseases are known to result from an inability of a patient's cells to produce a specific gene product. The present invention making genetically altered stem cells that can be used to produce cells with homozygous MHC alleles for transplantation that are genetically modified to synthesize enhanced amounts of a gene product required by the transplant recipient. For example, hematopoietic stem cells that are genetically altered to produce and secrete adenosine deaminase can be prepared for transplant to a patient suffering from adenosine deaminase deficiency. The methods of the present invention permit production of such cells without the use of recombinant retrovirus, which can insert at a site in the genomic DNA that disrupts normal growth control and causes neoplastic transformation.

Stem cells can also be genetically modified by introduction of a gene that causes the cell to die. The gene can be put under control of in inducible promoter. If for any reason the transplanted cells react in a in a way that can harm the recipient, expression of the suicide genes can be induced to kill the transplanted cells. Use of inducible suicide genes in this manner is known in the art. Suitable suicide genes include genes encoding HSV thymidine kinase and cytodine deaminase, with which cell death is induced by gancyclovir and 5-fluorocytosine, respectively.

The cells may be modified to knockout one or more histocompatibility antigen alletes, e.g., MHC alleles such that only one set remains. This leads to an underexpression of the MHC genes, but a phenotype effective in reducing the complexity of the MHC serotype and effective in producing cells capable of otherwise functioning and useful in the treatment of disease. Alternatively, homozygosity can be engineered into the cell lines by the targeted introduction of the appropriate alleles to the nonhomologous set, to result in homozygosity. In addition, the chromosome carrying the MHC genes can be removed from cells by laser ablation and a chromosome carrying the identical chromosome as remains in the cell can be added by microsome-mediated chromosome transfer, or by other techniques known in the art.

The present invention is by no means limited to the foregoing examples of genetic alterations. Persons skilled in the art will be able to identify numerous other ways by which stem cells produced according to the present invention can be genetically modified to enhance their utility.

### Preparing pluripotent stem cells:

Stem cells are present in the earliest stages of embryo formation. Embryonic stem cells (ES cells) are undifferentiated stem cells that are derived from the inner cell mass (ICM) of a blastocyst embryo.

According to the method of claim 57, the inner cell mass cells are of a blastocyst obtained by method comprising: (a) activating oocytes; (b) exposing the activated oocytes to chemical treatment that inhibits extrusion of the second polar body; and (c) culturing the embryo *in vitro* under conditions resulting in formation of a blastocyst. Further, according to claims 1 and 35 the stem cell bank comprises a plurality of human parthenogenetic stem cell lines.

Stem cells of the invention are derived from parthenogenetically derived blastocysts only.

A detailed method for preparing human ES cells is also described in Thomson's U.S. Patent No. 6,200,806, "Primate Embryonic Cells," issued March 13, 2001, disclosed for reference only.

Methods for growing human ES cells and maintaining them in an undifferentiated state without culturing them on a layer of feeder cells have also been described (Xu et al., Nature Biotechnology, 2001, 19:971-4).

Feeder-free culture of stem cells can reduce the risk of contamination of the cells by pathogens that may reside in the feeder cells.

### Generating differentiated cells:

Stem cells are widely regarded as an abundant source of pluripotent cellular material that can be directed to differentiate into cells and tissues that are suitable for transplantation into patients in need of such cell and tissue transplants. ES cells appear to have unlimited proliferative potential and are capable of differentiating into all of the specialized cell types of a mammal, including the three embryonic germ layers (endoderm, mesoderm, and ectoderm), and all somatic cell lineages and the germ line. Using known methods, ES cells can be cultured under conditions in which they differentiate into multipotent stem cells such as hematopoietic or neuronal stem cells. Alternatively, ES cells can be cultured under conditions in which they differentiate into a terminally differentiated cell type such as a cardiac muscle cell. Pluripotent stem cells homozygous for MHC alleles produced by the methods of the present invention can be cultured using methods and conditions known in the art to generate cell lineages that differentiate into many, if not all, of the cell types of the body, for transplant into human patients in need of such transplants. Such stem cells having one or more homozygous MHC alleles can differentiate into cells selected from the group consisting of immune cells, neurons, skeletal myoblasts, smooth muscle cells, cardiac muscle cells, skin cells, pancreatic islet cells, hematopoietic cells, kidney cells, and hepatocytes. For example, methods have been described by which ES cells are induced to differentiate in vitro into cardiomyocytes (Paquin et al., Proc. Nat. Acad. Sci. (2002) 99:9550-9555), hematopoietic cells (Weiss et al., Hematol. Oncol. Clin. N. Amer. (1997) 11(6): 1185-98; also U.S. Patent No. 6,280,718), insulin-secreting beta cells (Assady et al., Diabetes (2001) 50(8):1691-1697), and neural progenitors capable of differentiating into astrocytes, oligodendrocytes, and mature neurons (Reubinoff et al., Nature Biotechnology (2001) 19:1134-1140; also U.S. Patent No. 5,851,832).

Novel screening methods that make use of gene trapped cell lines and provide means for efficiently identifying combinations of biological, biochemical, and physical agents or conditions that induce stem cells to differentiate into cell types useful for transplant therapy, and for preparing and isolating specific differentiated cell types, are described in co-owned and co-pending U.S. Application No. 10/227,282, filed August 26, 2002,

In a useful embodiment of the present invention, a stem cell bank is produced that comprises hematopoietic stem cells homozygous for MHC antigens. A method for inducing the differentiation of pluripotent human embryonic stem cells into hematopoietic cells useful for transplant according to the present invention is described in U.S. Patent No. 6,280,718, "Hematopoietic Differentiation of Human Pluripotent Embryonic Stem Cells," issued to Kaufman et al. on August 28, 2001 .

The method disclosed in the patent of Kaufman et al. comprises exposing a culture of pluripotent human embryonic stem cells to mammalian hematopoietic stromal cells to induce differentiation of at least some of the stem cells to form hematopoietic cells that form hematopoietic cell colony forming units when placed in methylcellulose culture.

Those skilled in the art will appreciate that, using currently available methodologies, the pluripotent stem cells produced by the methods of the present invention can also be used to generate tissues formed of two or more different cell types homozygous for a MHC allele, for transplant to a person in need of such a tissue transplant.

The pluripotent stem cells homozygous for MHC antigens that are generated according to the present invention, and the lines of differentiated cells obtained from these stem cells, are produced and isolated under Good Manufacturing Practices (GMP) conditions.

### Providing Histocompatible Transplants To People Needing Them:

The methods for generating stem cells and differentiated cells having homozygous MHC alleles described above provide effective solutions to many of the problems associated with obtaining cells for transplant that are histocompatible with a transplant recipient. However, de novo production of histocompatible cells and tissue for transplantation by parthenogenesis for each patient in need of transplant is time-consuming. The time required to prepare "customized" cells or tissue for transplantation having the same HLA antigens as the transplant recipient can be problematic when the health of the would-be recipient is rapidly deteriorating for want of a transplant. Therefore, one or more of the above-described methods for generating stem cells and differentiated cells having homozygous MHC alleles are used to produce a stem cell bank comprising many different lines of stem cells, each having a different combination of homozygous MHC alleles present in the population. When a patient is found to be in need of a particular type of cell transplant, a line of stem cells from the stem cell bank having homozygous MHC alleles matching those of the patient can be taken "off the shelf" and cultured under conditions causing them to differentiate into the type(s) of cells needed. The differentiated cells are then isolated using known methods, and are provided to the patient's physician for transplant.

Accordingly, the present invention includes the process of identifying the type of cells needed for transplant, and the blood type and HLA antigens of the transplant recipient, selecting stem cells from the stem cell bank that differentiate into the cell type needed and have homozygous HLA antigens that match those of the transplant recipient; culturing the stem cells under conditions in which they differentiate into the cell type needed; isolating the differentiated cells needed for transplant; and providing these to the patient's physician for transplant into the patient.

The differentiated cells for transplant produced by these methods are homozygous for at least one HLA antigen present on cells of the transplant recipient. Histocompatibility of the cells for transplant and the recipient increases as a function of the number of homozygous HLA antigens of the cells for transplant that match HLA antigens of the recipient. The greater the number of homozygous HLA antigens of the cells for transplant that match HLA antigens of the recipient, the longer the graft is expected to survive without being rejected. The cells for transplant provided by the invention will therefore have one, two, three, four, five, six, or more homozygous HLA antigens that match HLA antigens of the recipient. For example, cells for transplant produced by the present invention can have homozygous HLA-A, HLA-B, and HLA-DR antigens that match HLA antigens of the recipient. Alternatively, the cells for transplant produced by the present invention can have homozygous HLA-A, HLA-B, HLA-C, HLA-DR, HLA-DQ, and HLA-DP antigens that match HLA antigens of the recipient. The ability to select stem cells "off the shelf" to produce cells for transplant having a relatively high number of homozygous HLA antigens that match those of a prospective transplant recipient depends on the size and complexity of the stem cells bank. A stem cell bank containing from 100,000 to 200,000 different stem cell lines, each having a different combination of homozygous HLA-A, HLA-B, and HLA-DR antigens, is required in order to be able to provide cells with homozygous HLA-A, HLA-B, and HLA-DR antigens that match the corresponding HLA antigens of a large percentage of people in a diverse population such as that of North America. Use of cells from individuals with blood type O can avoid rejection based on ABO blood type; but there would have to be two versions of each cell type in the stem cell bank in order to provide matches to the Rh(+) and Rh(-) blood types. Accordingly, a stem cell bank containing several hundred thousand stem cell lines can be expected to provide "off the shelf" stem cells that can be used to generate differentiated cells needed for transplant that have homozygous HLA-A, HLA-B, and HLA-DR antigens matching those of a person in need of such a transplant.

The stem cell bank of the present invention contains lines of pluripotent human stem cells, each having a specific combination of one or more homozygous HLA antigens. The lines of stem cells that can be used to generate cells or tissue suitable for transplant can be lines of human ES cells. The stem cell lines can also be pluripotent, partially differentiated stem cells such as myoblasts, hematopoietic stem cells, neuronal precursor cells, and endothelial cell precursor cells.

### Therapeutic cell transplantation:

Using the methods of the present invention, a line of pluripotent stem cells can be selected from a bank of such stem cells that are homozygous for one or more histocompatibility antigen alleles, i.e.

MHC alleles that match an MHC allele of a patient in need of transplant. For example, the stem cells can have homozygous HLA-A, HLA-B, and HLA-DR alleles that match HLA-A, HLA-B, and HLA-DR alleles of the patient. The stem cells are cultured ex vivo under conditions in which they are induced to differentiate into partially or fully differentiated cell types that are suitable for transplant and have homozygous MHC alleles that match MHC alleles of the patient in need of the transplant.
The partially or fully differentiated cells needed for transplant are isolated from other cell types, e.g., using antibody-based separation methods such as cell sorting or immunomagnetic beads, and antibodies that are specific for one or more differentiation antigens on the surface of the cell type needed for transplant.

The isolated partially or fully differentiated cells are then administered by transplantation to the patient using known methods. Methods for transplantation of epidermal cells, hematopoietic stem cells, Islet of Langerhans cells, chondrocytes, hepatocytes, myoblasts, neural cells, and endothelial cells are reviewed by Inverardi et al. (Transplantation Biology, Cellular and Molecular Aspects, Chapter 56, 1996, ed. by Tilney et al., Lippincott-Raven, Philadelphia, PA). The method to be used to transplant or engraft cells to a patient is recognized as depending on the type of cells to be transplanted, and on the pathology of the patient.

### Cells Homozygous for Recessive Disease-Causing Genes:

Recessive alleles responsible for genetically inherited diseases are endemic in the population. If cells of people carrying a recessive disease-causing gene are used to produce stem cells having homozygous HLA alleles from embryos generated by parthenogenesis
there is a relatively high likelihood that some of the stem cell lines obtained also be homozygous for the recessive disease-causing gene. The stem cell lines produced by the methods of the present invention can therefore be screened to identify those which are homozygous for a recessive disease-causing gene. Such screening can be carried out using known methods. For example, DNA sequences of the cells can be amplified by the polymerase chain reaction (PCR) and analyzed by DNA sequencing, restriction enzyme cleavage, or by hybridization to an array of oligomers, e.g., on a microchip. Examples of recessive-disease causing genes to be screened for include, but are not limited to, of recessive genes causing the following conditions:
Adenosine deaminase deficiency
Albinism
Adenylosuccinate lyase deficiency
Alpha-1 antitrypsin deficiency
Cystic Fibrosis
Friedreich's ataxia
Gaucher's disease
hypercholesterolemia
Alzheimer's Disease
Autoimmune polyendocrinopathy candidiasis-ectodermal dystrophy
AID - deficiency of activation-induced cytidine deaminase
Ataxia-telangiectasia
CD3-epsilon deficiency (causes SCID)
CD3-gamma deficiency (causes SCID)
chronic granulomatous disease - deficiency of p47^{phox}
Phenylketonuria - Phenylalanine hydroxylase (PAH) deficiency
Tetrahydrobiopterin deficiencies:
   GTP cyclohydrolase I (GTPCH) deficiency
   6-Pyruvoyl-tetrahydropterin synthase (PTPS) deficiency
   Dihydropteridine reductase (DHPR) deficiency
   Pterin-4a-carbinolamine dehydratase (PCD) deficiency
Janus Kinase 3 (JAK3) deficiency (causes SCID)
Hereditary fructose intolerance
Porphyria (one of the six forms is caused by a recessive gene)
Sickle Cell Anemia
Tay Sachs syndrome
Thalassemia
Wilson's disease
Xeroderma pigmentosum
Zeta-chain-associated protein kinase deficiency (causes SCID)

The pluripotent stem cell lines having a homozygous recessive disease-causing gene that are produced by the methods of the present description are highly useful. They can be cultured under conditions in which they differentiate into cell types related to manifestation of the disease phenotype. Such cells having a homozygous recessive disease-causing gene are useful for basic research directed to studying the disease phenotype ex vivo. They can also be implanted into experimental animals (e.g., immunodeficient animals), for study of their metabolic activities in vivo. Persons skilled in the art would recognize that studies in which such cells are genetically modified can be useful for gaining understanding of the disease phenotype. Such cells having a homozygous recessive disease-causing gene can also be used in drug discovery; e.g., in screening for drugs or other therapies that will treat or cure the disease caused by the recessive gene.

In order to further illustrate the invention and its preferred embodiments, the following examples are provided. These examples are intended to be exemplary and in no way limitative of the scope of the present invention, which is defined by the claims.

### EXAMPLE 1

### Production of Parthogenic Primate Primordial Stem Cells (PPSC's)

### Materials and Methods

1. Cynomolgous Monkey (Macaca fascicularis) were superovulated using a single injection of 1000 IU of pregnant mare's serum gonadrophin (PMSG) and 500 IU of human chorionic gonadoprophin (hCG) four days later.
2. Ovaries were retrieved by laparotomy and oocytes dissected from the follicles and placed in maturation media 36 to 48 hrs after (hCG). Maturation media consisted of medium-199 (Gibco BRL) with Earle's balanced salt solution supplemented with 20% fetal bovine serum, 10 IU/ml of PMSG, 10 IU/ml of hCG, 0.05 mg/ml of penicillin G and 0.075 mg/ml of steptomycin sulfate (Hong, 1999).
3. Oocyte activation
   After 40 hrs in maturation, metaphase II eggs were placed in 10 micromoles of lonomycin followed incubation in 200 mM 6-DMAP (dimethylaminopurine) for 3 to 4 hrs.
4. Embryo culture. Commercially available embryo culture media 'Cooks' was used (modified SOF). Embryos were cultured with a co-culture of mitotically inactivated mouse embryonic fibroblasts as feeder layer.
5. Isolation of inner cell mass
   a) Upon development to blastocyst, embryos were placed in a buffered solution of 0.3% pronase for 2 minutes to digest zona pellucida
   b) Blastocyts were then rinsed in buffered solution and moved to solution of G1 culture media and polyclonal antibodies (antihuman whole serum) 1:3 dilution for 30 minutes.
   c) Embryos were rinsed 5 times in a buffered solution.
   d) Embryos were moved into a solution of G1 culture media and guinea pig complement 1:3 dilution for 30 minutes.
   e) Remaining of the embryos (dead trophoblast cells and ICM) wee rinsed 5 times in buffered solution the Inner Cell Mass (ICM) was isolated and placed on top of a mouse embryonic fibroblast feeder layer for isolation and growth of Primordial Stem Cells (PSC's).

### Results

We have obtained 450 eggs total, after maturation, 224 were still at germinal vesicle stage (GV = no maturation), 79 were dead, 56 were at metaphase one (MI) and 91 at metaphase two (MII).

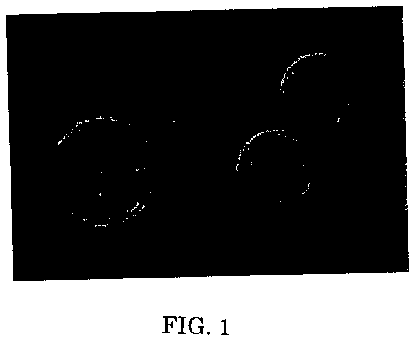

We have parthenogenically activated all of them. As expected, there was no cleavage on the GV group, 32% cleavage on the MI and 57% on the MII. When put in culture, 7 embryos developed to the blastocyst stage (See Figure 1).

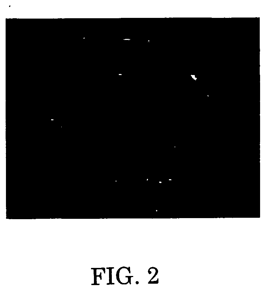

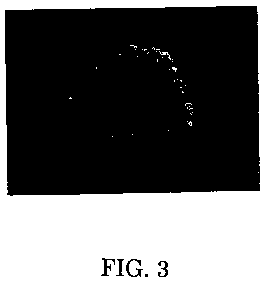

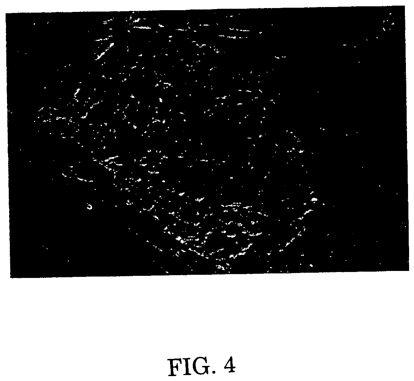

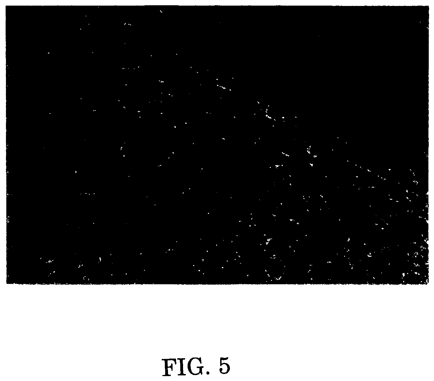

After attempting to establish ES-like culture cells, four Inner cell masses attached nicely one differentiated immediately, and out of the three remaining, one cell line was obtained. This cell line is called Cyno 1 (Fig. 2). This cell line before and after immunosurgery is shown in Figures 3 and 4. Figure 5 shows the Cyno 1 Cell line five days after plating.

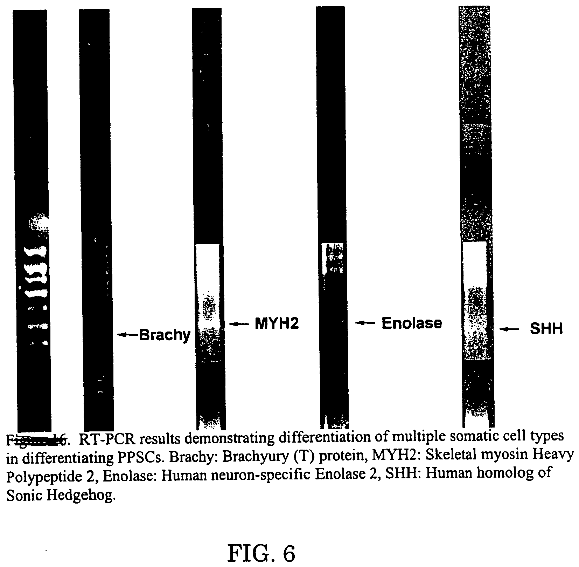

Figure 6 shows the Cyno 1 cell line growing on top of a mouse fibroblast feeder layer. These cells show typical morphology of pluripotent-embryonic-cells such as small nuclear cytoplasmic ration and the presence of cytoplasmic granules.

These cells were maintained in an undifferentiated state for a period of months. This is evidenced by screening of such cells after prolonged culturing for the expression of a cell marker characteristic of undifferentiated cells, Alkaline Phosphatase. As expected, cells were positive on passage 3 and on passage 5.

The fact that these cells maintain their pluripotency is also shown by their spontaneous differentiation into many differentiated cell types after being placed in tissue culture in the absence of a feeder layer. In the days following, the cells were observed to differentiate into cuboidal epithelium, fibroblasts, beating myocardial cells and other cells. Two colonies of beating myocardial cells wee observed in one well of a 4-well tissue culture plate.

To determine whether differentiated cells of various somatic cell lineages were observed from the differentiating PPSC's, we extracted mRNA from differentiated cell cultures, performed RT-PCR, using human sequence primers specific for various differentiated cell types. As shown in figure 6, transcripts of a predicted size for the mesodermally-derived transcripts brachyury and skeletal muscle myosin heavy polypeptide 2 were observed. The transcript sonic hedgehog essential for endoderm development was observed. In addition, the neuron-specific ectoderm marker enolase was observed as well as keratin (not shown) as markers of ectodermally derived cells. These PCR products were not observed in the mouse feeder layer controls or in the absence of reverse transcriptase.

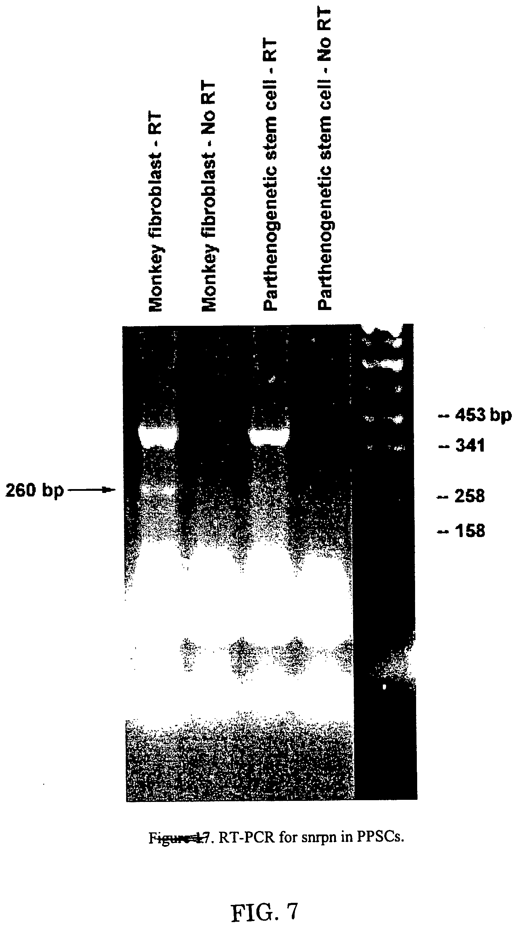

To establish that the imprinting status of parthogenetic PPSC's different than that of di-parental PPSCs we looked at the expression of several imprinted genes. Genes that are mono-allelically expressed from the paternal allele, would not be expected to be expressed in parthogenetic cells, as these cells are derived exclusively from the maternal genome. The *Snrpn* gene is mono-allelically expressed from the paternal allele in mouse blastocyst inner cell mass [Szabo,PE and Mann, JR; Genes & Development 9:3097-3108 (1995)]. We looked at the expression of this gene in the parthogenetic *Macaca facicularis* PPSCs and found that the express was undetectable by RT-PCR, whereas under identical conditions, this gene is readily detected in fibroblast cell cultures from the same species. The *Snrpn* gene is expected to be expressed in diparental PPSCs, as these cells contain a paternal allele. In Figure 7, the expected size RT-PCR product for the *Snrpn* gene is 260 bp.

### EXAMPLE 2

### Stable Engraftment of Homozygous Fibroblasts in Histocompatible are Non-Histocompatible Cynomolqous Recipients

Connective tissue fibroblasts are generated from the cyno-1 stem cell line described above which are labeled with green flourescent protein (GFP) gene. This cell line is homozygous as evidenced by the portion of a single allete of 225 basepairs using a primer set specific for DQBlu6011-17. These cells are propogated in vitro until several million cells are obtained.

Thereafter, approximately a million labeled connective fibroblasts are transplanted into histocompatible cynomolyus monkey recipients, and non-histocompatible cynomolgous controls. Each monkey is transplanted with a million labeled cells administered by injection in the upper arm at for different sites, in four equal parts.

The degree of engraftment of these engrafted labeled cells is assessed at three different times, at four weeks, six months and a year. Three of the four grafts are removed at three different times and the number of GFP labeled cells is determined in the histocompatible transplant recipients and controls. The number of GFP cells is compared for both groups.

Also, a histological examination is effected to look for any signs of lymphocyte infiltration and any signs of rejection.

### EXAMPLE 3

### Production of Homozygous Stem Cell Lines from Rabit Parthenogenically Activated Ooyctes

Rabbit ES cells were similarly obtained from parthenogenetic embryos. Specifically, rabbit oocytes were obtained from superovulating rabbits and were acttuated using ionomycin and DMAP. This resulted in blastocystes, the inner cell masses of which were transferred to fibroblast factor layer. This in turn resulted in the production of rabbit ES cell lines which stained positive for characteristic embryonic antigens and which gave rise to various differentiated cell types when removed from the front layer.

More specifically, true rabbit ES cell lines morphlogically looked like ES cells and differentiated into into all three germ cell linenages. Among the cell types that observed from this cell line were myocordial, vascular endothalial, neuronal, and hemotopoiath cell lineages.

### EXAMPLE 4

### Protection of Homozygous Stem Cell Lines from Human Parthenogenically Activated Oocytes

### Production of Autologous Cells by Parthenogenetic Activation of Oocytes

Oocytes from three volunteers were used for parthenogenetic activation. The donors were induced to superovulate by 11 days of low dose (75 IU bid) gonadotropin injections prior to hCG injection. A total of 22 oocytes were obtained from the donors 34 hours after HCG stimulation, and were activated at 40-43 h after hCG stimulation.

The oocytes were activated on day 0, using the ionomycin/DMAP activation protocol described above. Twelve hours after activation, 20 oocytes (90%) developed one pronucleus and cleaved to the two-cell to four-cell stage on day 2. On day 5 of culture, evident blastocoele cavities were observed in six of the parthenotes (30% of the cleaved oocytes) though none of the embryos displayed a clearly discemible inner cell mass. The results of parthenogenetic activation of the human oocytes are summarized in Table 4.

**Table 4, Parthenogenetic Activation of Human Oocytes**

| Donor | No. of Oocytes | Pronucleus (%)^{a} | Cleaved (%)^{a} | Embryos with blastocoele Cavity (%)⁵ |
|---|---|---|---|---|
| 1 | 5 | 4(80) | 4(80) | 0 |
| 2 | 14 | 13(93) | 13(93) | 4(31) |
| 6 | 5 | 3 | 3(100) | 2(67) |
| Total | 22 | 20(90) | 20(90) | 6(30) |

| | | | | |
|---|---|---|---|---|
| ^{a} As a percentage of activated oocytes. ^{b} As percentage of cleaved oocytes. | | | | |

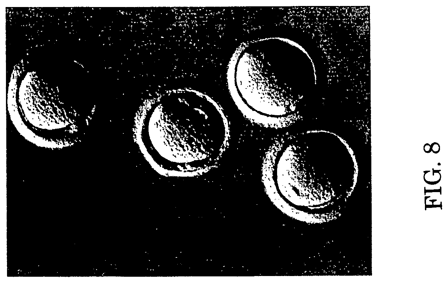

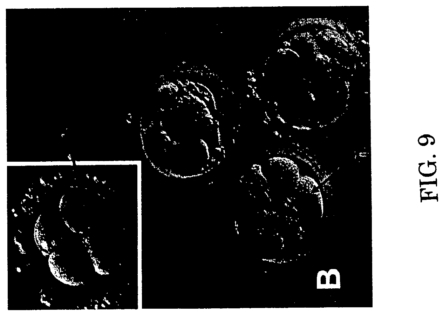

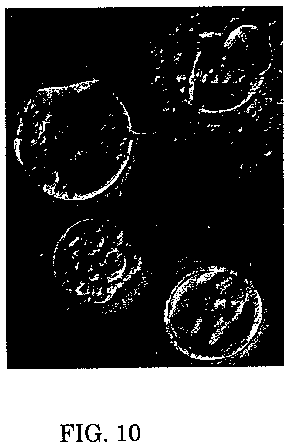

Figure 8 shows MII oocytes at the time of retrieval. Figure 9 shows four-to six-cell embryos 48 h after activation. Distinguishable single-nucleated blastomeres (labeled "n" in Fig. 6) were consistently observed. Figure 10 shows embryos with blastocoele cavities (arrows) that were detected on day 6 and maintained in culture until day 7. The scale bars for Figures 6 - 8 = 100 µm.

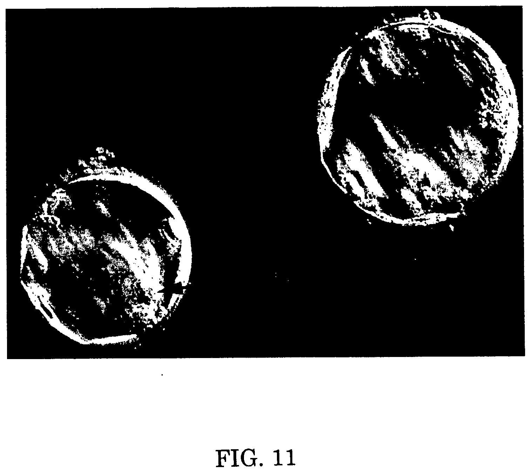

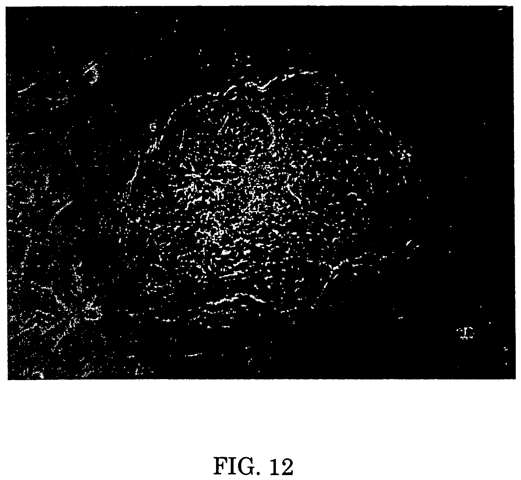

In a study similar to the one described above, human oocytes were activated using the ionomycin/DMAP activation protocol and were cultured *in vitro.* One of the activated embryos developed a pronucleus, cleaved, formed a blastocoele cavity, and then developed into a blastocyst having an inner cell mass, shown in Figure 11. The inner cell mass was isolated and plated on mouse feeder layers as described (Cibelli, J.B., et al. 2002. Parthenogenetic stem calls in non-human primates. Science 295: 819). The cultured ICM cells increased in number over the first week, and cells indistinguishable from human embryonic stem cells were observed. These grew in close association as a colony with a distinct boundary, as shown in Figure 12; they had a high nuclear-to-cytoplasmic ratio, prominent nucleoli, and were observed to differentiate *in vitro* into multiple differentiated cell types.

### EXAMPLE 5

### Production of Homozygous Stem Cell Lines from Mouse Parthenogenically Activated Oocytes

Using substantially the same methods described in the present application, another research group, Lin et al., Stem Cells 21:152-161 (2003) , generated stem cell lines from unfertilized mouse metaphase II oocytes. These oocytes were activated by 5 minute exposure to 5mm calcium ionophore (ionomycin) followed by a 3 hour exposure to 6-methyldiaminopurine (DMAP). Those stem cell lines were characterized as stem cell lines based on their expression of characteristic embryonic antigens (SSEAs, OCT-4, alkaline phosphatase and telomerase) and their pluripotency (give rise to ectodermal, endodermal and mesodermal cell types).

Specifically, activated, unertilized oocytes from F1 hybrid mice (H-2-B/D0 were used to establish those stem cell lines homozygous for H-2-B and H-2-D respectively. The stem cell lines appeared karyotypically normal. When cultured in vitro in the pressures of specific growth factors, these cell lines gave rise to ectodermal, mesodermal, and enodermal cell types. Histological examination of cultures revealed cells having the morphology of neuronal cells and hemotopviette lineages (lymphocytes, monocytes and erythrocytes).

Further, when these cell lines were implanted in the kidney of syngenetic F1 mice they similarly resulted in teratomas that comprised cells of all three germ layers. The teratemas when histologically examined showed evidence of hair follicles, thyroid glands, lung epithelium and connective tissue.

### CONCLUSIONS

The results in the foregoing examples provides proof of principle, namely that homozygous stem cell lines may be generated from parthenogenically activated embryos, and used to produce differentiated cell types for cell therapy. More specifically, the present instruction provides methods for making libraries or banks of stem cell lines that are homozygous for specific MHC alleles. Thereby, a bank of cells is available which can be used to produce differentiated cells which are histocompatible for a wide range of transplant recipients. This is feasible with a relatively few number of stem cell lines given that certain HLA haplotyes are expressed with relatively high frequency in the human population.

These differentiated cells should be well tolerated and be stably engrafted given their antigenic expression relative to the transplant recipient. Also, stem cell lines derived from parthenogenically activated oocytes eliminate certain ethical issues with therapeutic cloning, namely a viable embryo (capable of giving rise to an offspring) is never obtained or destroyed. These cells are useful for treating any condition wherein cell or tissue transplantation is therapeutically desirable, e.g. immune deficiencies, age-related deficiencies, cancer, autoimmune disorder, organ deficiencies, disease, or injury, burn, malignancy, cell proliferation disorders, hemotopoietic disorders, e.g. blood malignancy such as non-Hodgkins lymphoma, leukemia, inflamatory disorders, connective tissue disorder, dermatological disorder, ischemia, stroke, neurological disorders and the like. The present cell banks on particularly well suited for treating acute disease, particularly when there is not sufficient time to do therapeutic cloning. For example, those cells are useful in obtaining differentiated cells for treatment of conditions where the patient is near death, eg., sepsis, stroke and other conditions where cell therapy is urgently needed. Also, the invention provides means for having cells on hand that express desired therapeutic polypeptides which are histocompatible.

## Claims

1. A stem cell bank comprising a library or plurality of human parthenogenetic stem cell lines, each of which is homozygous for at least one MHC allele present in a human population, wherein each member of said plurality of stem cell lines is homozygous for a different set of MHC alleles relative to the remaining members of the plurality of stem cell lines.

2. The stem cell bank of claim 1 which comprises at least five different stem cell lines, each member homozygous for a different combination of histocompatibility or MHC antigen alleles.

3. The stem cell bank of claim 1 which comprises at least ten different stem cell lines each member homozygous for a different combination of histocompatibility or MHC antigen alleles.

4. The stem cell bank of claim 1 which comprises at least about 100 to 1000 different parthenogenetic stem cell lines.

5. The stem cell bank of claim 1 which comprises human parthenogenetic stem cell lines that are at least homozygous for the 10 most frequent MHC alleles in the human population.

6. The stem cell bank of claim 1 which comprises human parthenogenetic stem cell lines that are homozygous for at least the 15 most frequent MHC alleles in the human population.

7. The stem cell bank of claim 1 wherein all of the stem cell lines are O-negative.

8. The stem cell bank of claim 5 wherein the library includes human parthenogenetic stem cells that are homozygous for one or more of the following MHC alleles: HLA-A1, HLA-A2, HLA-A3, HLA-A24, HLA-A11, HLA-A28, HLA-A29, HLA-A32, HLA-B15, HLA-B5, HLA-B7, HLA-B8, HLA-B12, HLA-B14, HLA-B18, HLA-B35, and HLA-B40.

9. The stem cell bank of claim 8 wherein all of said human parthenogenetic stem cell lines are O-negative.

10. The cell bank of claim 1 which includes at least one human parthenogenetic stem cell line which is rendered homozygous for an MHC allele by removal of one or more MHC alleles by knockout or RNA interference.

11. The cell bank of claim 5 which includes stem cell lines which are homozygous for at least one of the following HLA-A, HLA-B and HLA-DR haplotype combinations: 1, 7, 2; 1, 8, 3; 2, 14, 1; 2, 35, 4, 2, 35, 8; 2, 44, 4; 3, 7, 2; 3, 7, 4; 3, 7, 8; 3, 35, 1; 31, 51, 4; and 32, 14, 7.

12. The stem cell bank of claim 11 which contains cell lines homozygous for all of said HLA-A, HLA-B and HLA-DR haplotype combinations.

13. The stem cell bank of claim 11 or 12 which is O-negative.

14. The stem cell bank of claim 10 in which the cells are rendered homozygous by the removal of one set of MHC alleles by knockout or RNA interference and the addition of another homozygous set of MHC alleles.

15. The stem cell bank of claim 1, which contains a plurality of lines of stem cells that can differentiate into hematopoietic stem cells.

16. The stem cell bank of claim 1, which contains a plurality of lines of hematopoietic stem cells.

17. The stem cell bank of clam 1, which contains a plurality of lines of stem cells that can differentiate into vascular endothelial precursor cells.

18. The stem cell bank of claim 1 which contain a plurality of stem cells that can differentiate into myocardial cells.

19. The stem cell bank of claim 1, which contains a plurality of stem cells that can differentiate into hepatic cells.

20. The stem cell bank of claim 1, which contains a plurality of stem cells that can differentiate into pancreatic beta cells.

21. The stem cell bank of claim 1, which contains a stem cell line homozygous for an MHC allele selected from HLA-A, HLA-B, HLA-C, HLA-DR, HLA-DQ, and HLA-DP, wherein the cells are of the O-negative blood group.

22. The stem cell bank of claim 1, which contains a stem cell line homozygous for the MHC alleles encoding HLA-A, HLA-B, and HLA-DR.

23. The stem cell bank of claim 22, wherein the stem cell line is of the O-negative blood group.

24. The stem cell bank of claim 1, which contains lines of diploid stem cells in which all of the MHC alleles are homozygous.

25. The stem cell bank of claim 1, which contains at least one human parthenogenetic stem cell line that comprises DNA that is genetically modified relative to the human donor from which the cell line is derived.

26. The stem cell bank of claim 25, in which the DNA or the genetically altered stem cell line is modified by adding, modifying, substituting or deleting one or more DNA sequences.

27. The stem cell bank of claim 25, wherein the DNA of the genetically altered stem cell line is modified so as to obtain, increase, decrease, inhibit, or otherwise modify, the expression of a gene that is native to, or introduced into, cells of the at least one cell line, relative to expression of said gene in a control cell without the genetic modification.

28. The stem cell bank of claim 25, wherein the DNA of the genetically altered stem cell line is modified by homozygous recombination.

29. The stem cell bank of claim 25, wherein the DNA of the genetically altered stem cell line is altered to prevent the expression of a gene encoding an antigenic protein that elicits an immune response contributing to rejection.

30. The stem cell bank of claim 25, wherein the DNA of the genetically altered stem cell line is altered to express a gene that inhibits the immune rejection of that cell.

31. The stem cell bank of claim 28, wherein the DNA of the genetically altered stem cell line is modified so as to inhibit production of at least one HLA antigen by cells of said cell line.

32. The stem cell bank of claim 31, wherein the at least one HLA antigens is selected from HLA-A, HLA-B, HLA-C, HLA-DR, HLA-DQ, and HLA-DP.

33. The stem cell bank of claim 28, wherein the DNA of the genetically altered stem cell line is modified so as to inhibit production of Beta2-microglobulin.

34. The stem cell bank of claim 28, wherein the DNA of the genetically altered stem cell line is altered by replacing a non-homozygous MHC allele with one that is homozygous.

35. An *in vitro* method for preparing a cell or tissue for transplant, comprising preparing cells or tissue for transplantation that are homozygous for at least one HLA allele in a person in need of such a transplant, comprising:
(a) identifying the MHC alleles of a person in need of a transplant (the recipient);
(b) obtaining stem cells which are homozygous for the MHC alleles identified in (a) from a stem cell bank comprising a plurality of human parthenogenetic stem cell lines homozygous for at least one MHC allele of the transplant recipient; and
(c) generating cells or tissue suitable for transplant from said stem cells.

36. The method of claim 35 wherein said stem cell bank comprises at least 10 different human parthenogenetic stem cell lines, wherein each of said stem cell lines are homozygous for a different combination of HLA alleles relative to the other stem cell lines.

37. The method of claim 35 wherein said stem cell bank comprises at least 15 different human parthenogenetic stem cell lines wherein each of said stem cell lines are homozygous for a different combination of HLA alleles relative to the other human stem cell lines in the cell bank.

38. The method of claim 35 wherein said stem cell bank comprises at least about 100 to 1000 human parthenogenetic stem cell lines each homozygous for a different combination of HLA alleles relative to the other human stem cell lines in the cell bank.

39. The method of claim 35 wherein said human stem cell bank comprises cells which are homozygous for one of the following HLA serotypes: HLA-A1, HLA-A3, HLA-A11, HLA-A15, HLA-A22, HLA-A27, HLA-A28, HLA-A29, HLA-A32, HLA-B5, HLA-B7, HLA-B8, HLA-B 12, HLA-B 17, HLA-B 18, HLA-B35 and HLA-B40.

40. The method of claim 35 wherein said human stem cell bank comprises stem cells which, are homozygous for at least one of the following HLA-A, -B or -DR haplotypes : 1, 7, 2; 1, 8, 3; 2, 14, 1; 2, 35, 4; 2, 35, 8; 2, 44, 4; 3, 7, 2; 3, 7, 4; 3, 7, 8; 3, 35, 1; 31, 51, 4; and 32, 14, 7.

41. The method of claim 39 or 40 wherein said cell banks are O-negative.

42. The method of claim 35, wherein step b comprises obtaining stem cells that can differentiate into hematopoietic stem cells.

43. The method of claim 35, wherein step b comprises obtaining hematopoietic stem cells from the stem cell bank.

44. The method of claim 35, wherein step b comprises obtaining stem cells homozygous for an MHC allele selected from HLA-A, HLA-B, HLA-C, HLA-DR, HLA-DQ, and HLA-DP.

45. The method of claim 44, wherein the MHC alleles encode HLA-A, HLA-B, and HLA-DR.

46. The method of claim 35, wherein step b comprises obtaining diploid stem cells derived from embryos produced by parthenogenesis.

47. The method of claim 46, comprising obtaining diploid stem cells in which all of the MHC alleles are homozygous.

48. The method of claim 35, wherein step b comprises obtaining stem cells having DNA that is genetically modified relative to the DNA of the human donor from which the stem cells are derived.

49. The method of claim 48, comprising obtaining genetically altered stem cells, the DNA of which is modified by adding, modifying, substituting, or deleting one or more DNA sequences.

50. The method of claim 48, comprising obtaining genetically altered stem cells, the DNA of which is modified so as to obtain, increase, decrease, inhibit, or otherwise modify, the expression of a gene that is native to or introduced into said cells, relative to expression of said gene in a control cell without the genetic modification.

51. The method of claim 48, comprising obtaining genetically altered stem cells, the DNA of which is modified by homologous recombination.

52. The method of claim 48, comprising obtaining genetically altered stem cells, the DNA of which is altered to prevent the expression of a gene encoding an antigenic protein that elicits an immune response contributing to rejection.

53. The method of claim 51, comprising genetically altered stem cells, the DNA of which is modified so as to inhibit production of at least one HLA antigen by cells of said cell line.

54. The method of claim 53, wherein the at least one HLA antigen is selected from HLA-A, HLA-B, HLA-C, HLA-DR, HLA-DQ, and HLA-DP.

55. The method of claim 51, comprising genetically altered stem cells, the DNA of which is modified so as to inhibit production of Beta2-microglobulin.

56. The method of claim 51, comprising genetically altered stem cells, the DNA of which is altered by replacing a non-homozygous MHC allele with one that is homozygous.

57. A method for producing human stem cells homozygous for at least one MHC allele present in a human population, comprising:
(a) activating oocytes;
(b) exposing the activated oocytes to chemical treatment that inhibits extrusion of the second polar body;
(c) culturing the parthenogenetic embryo in vitro under conditions resulting in formation of a blastocyst;
(d) culturing inner cell mass cells of the blastocyst in vitro under conditions resulting in production of stem cells; and
(e) screening to identify stem cells homozygous in a particular MHC allele.

58. The method of claim 57 wherein said method is repeated with oocytes from different donors and a bank or library of different stem cell lines which are homozygous for different MHC alleles relative to the other members in the group are obtained.

59. The method of claim 58 wherein said stem cell bank includes stem cell lines which are homozygous for one or more of the following HLA serotypes: HLA-A1, HLA-A3, HLA-A11, HLA-A15, HLA-A22, HLA-A24, HLA-A28, HLA-A29, HLA-A32, HLA-B5, HLA-B7, HLA-B8, HLA-B 12, HLA-B 17, HLA-B 18, HLA-B35 AND HLA-B40.

60. The method of claim 58 wherein said stem cell bank includes stem cell lines which are homozygous for at least one of the following HLA-a, -B and -DR haplotypes: 1, 7, 2; 1, 8, 3; 2, 14, 1; 2, 35, 4; 2, 35, 8; 2, 44, 4 ; 3, 7, 2; 3, 7, 4; 3, 7, 8; 3, 35, 1; 31, 51, 4 and 32, 14, 7.

61. The method of claim 58, claim 59 or claim 60 wherein said stem cell lines are O-negative.

62. The method of claim 57, wherein the stem cells can differentiate into hematopoietic stem cells.

63. The method of claim 57, wherein the stem cells are homozygous for an MHC allele selected from HLA-A, HLA-B, HLA-C, HLA-DR, HLA-DQ, and HLA-DP.

64. The method of claim 63, wherein the MHC alleles encode HLA-A, HLA-B, and HLA-DR.

## Patentansprüche

1. Stammzellbank, umfassend eine Bibliothek oder eine Mehrzahl menschlicher parthenogenetischer Stammzelllinien, von denen jede homozygot für mindestens ein MHC-Allel ist, das in einer menschlichen Population vorhanden ist, wobei jedes Mitglied der Mehrzahl von Stammzelllinien für einen unterschiedlichen Satz von MHC-Allelen relativ zu den verbleibenden Mitgliedern der Mehrzahl von Stammzelllinien homozygot ist.

2. Stammzellbank nach Anspruch 1, die mindestens fünf verschiedene Stammzelllinien umfasst, wobei jedes Mitglied homozygot für eine unterschiedliche Kombination von Histokompatibilitäts- oder MHC-Antigen-Allelen ist.

3. Stammzellbank nach Anspruch 1, die mindestens zehn verschiedene Stammzelllinien umfasst, die jeweils homozygot für eine unterschiedliche Kombination von Histokompatibilitäts- oder MHC-Antigen-Allelen sind.

4. Stammzellbank nach Anspruch 1, die mindestens etwa 100 bis 1.000 verschiedene parthenogenetische Stammzelllinien umfasst.

5. Stammzellbank nach Anspruch 1, die menschliche parthenogenetische Stammzelllinien umfasst, die mindestens für die 10 häufigsten MHC-Allele in der menschlichen Population homozygot sind.

6. Stammzellbank nach Anspruch 1, die menschliche Stammzelllinien umfasst, die mindestens für die 15 häufigsten MHC-Allele in der menschlichen Population homozygot sind.

7. Stammzellbank nach Anspruch 1, wobei alle Stammzelllinien O-negativ sind.

8. Stammzellbank nach Anspruch 5, wobei die Bibliothek menschliche parthenogenetische Stammzellen umfasst, die homozygot für eines oder mehrere der folgenden MHC-Allele sind: HLA-A1, HLA-A2, HLA-A3, HLA-A24, HLA-A11, HLA-A28, HLA-A29, HLA-A32, HLA-B15, HLA-B5, HLA-B7, HLA-B8, HLA-B12, HLA-B14, HLA-B18, HLA-B35 und HLA-B40.

9. Stammzellbank nach Anspruch 8, wobei alle menschlichen parthenogenetischen Stammzelllinien O-negativ sind.

10. Zellbank nach Anspruch 1, die mindestens eine menschliche parthenogenetische Stammzelllinie umfasst, die für ein MHC-Allel homozygot gemacht wird, indem ein oder mehrere MHC-Allele durch Knockout oder RNA-Interferenz entfernt werden.

11. Zellbank nach Anspruch 5, die Stammzelllinien umfasst, die für mindestens eine der folgenden HLA-A, HLA-B und HLA-DR-Haplotyp-Kombinationen homozygot sind: 1,7, 2; 1,8, 3; 2, 14, 1; 2, 35, 4; 2, 35, 8; 2, 44, 4; 3, 7, 2; 3, 7, 4; 3, 7, 8; 3, 35, 1; 31, 51, 4; und 32, 14, 7.

12. Stammzellbank nach Anspruch 11, die Zelllinien enthält, die für alle HLA-A-, HLA-B- und HLA-DR-Haplotyp-Kombinationen homozygot sind.

13. Stammzellbank nach Anspruch 11 oder 12, die O-negativ ist.

14. Stammzellbank nach Anspruch 10, in welcher die Zellen homozygot gemacht werden, indem ein Satz von MHC-Allelen durch Knockout oder RNA-Interferenz entfernt wird und ein weiterer homozygoter Satz von MHC-Allelen hinzugefügt wird.

15. Stammzellbank nach Anspruch 1, die eine Mehrzahl von Stammzelllinien enthält, die sich in hämatopoetische Stammzellen differenzieren können.

16. Stammzellbank nach Anspruch 1, die eine Mehrzahl von Linien von hämatopoetischen Stammzellen enthält.

17. Stammzellbank nach Anspruch 1, die eine Mehrzahl von Stammzelllinien enthält, die sich in vaskuläre endotheliale Vorläuferzellen differenzieren können.

18. Stammzellbank nach Anspruch 1, die eine Mehrzahl von Stammzellen enthält, die sich in Myokardzellen differenzieren können.

19. Stammzellbank nach Anspruch 1, die eine Mehrzahl von Stammzellen enthält, die sich in Leberzellen differenzieren können.

20. Stammzellbank nach Anspruch 1, die eine Mehrzahl von Stammzellen enthält, die sich in Pankreas-Beta-Zellen differenzieren können.

21. Stammzellbank nach Anspruch 1, die eine Stammzelllinie enthält, die homozygot für ein MHC-Allel ist, ausgewählt aus HLA-A, HLA-B, HLA-C, HLA-DR, HLA-DQ und HLA-DP, wobei die Zellen der O-negativen Blutgruppe angehören.

22. Stammzellbank nach Anspruch 1, die eine Stammzelllinie enthält, die homozygot für die für HLA-A, HLA-B und HLA-DR kodierenden MHC-Allele ist.

23. Stammzellbank nach Anspruch 22, wobei die Stammzelllinie der O-negativen Blutgruppe angehört.

24. Stammzellbank nach Anspruch 1, die Linien diploider Stammzellen enthält, in denen alle MHC-Allele homozygot sind.

25. Stammzellbank nach Anspruch 1, die mindestens eine menschliche parthenogenetische Stammzelllinie enthält, die DNA umfasst, die im Vergleich zu dem menschlichen Spender, von dem die Zelllinie stammt, genetisch modifiziert ist.

26. Stammzellbank nach Anspruch 25, wobei die DNA oder die genetisch veränderte Stammzelllinie durch Hinzufügen, Modifizieren, Ersetzen oder Löschen einer oder mehrerer DNA-Sequenzen modifiziert wird.

27. Stammzellbank nach Anspruch 25, wobei die DNA der genetisch veränderten Stammzelllinie modifiziert ist, um die Expression eines Gens zu erhalten, zu erhöhen, zu verringern, zu hemmen oder auf andere Weise zu modifizieren, das zu Zellen der mindestens einen Zelllinie nativ oder in diese Zellen eingeführt ist, bezogen auf die Expression des Gens in einer Kontrollzelle ohne die genetische Veränderung.

28. Stammzellbank nach Anspruch 25, wobei die DNA der genetisch veränderten Stammzelllinie durch homozygote Rekombination modifiziert ist.

29. Stammzellbank nach Anspruch 25, wobei die DNA der genetisch veränderten Stammzelllinie verändert wird, um die Expression eines Gens zu verhindern, das ein antigenes Protein kodiert, das eine Immunantwort hervorruft, die zur Abstoßung beiträgt.

30. Stammzellbank nach Anspruch 25, wobei die DNA der genetisch veränderten Stammzelllinie verändert wird, um ein Gen zu exprimieren, das die Immunabstoßung dieser Zelle hemmt.

31. Stammzellbank nach Anspruch 28, wobei die DNA der genetisch veränderten Stammzelllinie so modifiziert ist, dass sie die Produktion von mindestens einem HLA-Antigen durch Zellen der Zelllinie hemmt.

32. Stammzellbank nach Anspruch 31, wobei das mindestens eine HLA-Antigen aus HLA-A, HLA-B, HLA-C, HLA-DR, HLA-DQ und HLA-DP ausgewählt ist.

33. Stammzellbank nach Anspruch 28, wobei die DNA der genetisch veränderten Stammzelllinie so modifiziert ist, dass sie die Produktion von Beta2-Mikroglobulin hemmt.

34. Stammzellbank nach Anspruch 28, wobei die DNA der genetisch veränderten Stammzelllinie durch Ersetzen eines nicht homozygoten MHC-Allels durch ein homozygotes MHC-Allel verändert wird.

35. In-vitro-Verfahren zur Vorbereitung einer Zelle oder eines Gewebes für eine Transplantation, umfassend das Vorbereiten der Zellen oder Gewebe auf die Transplantation, die für mindestens ein HLA-Allel bei einer Person, die eine solche Transplantation benötigt, homozygot sind, umfassend:
(a) Identifizieren der MHC-Allele einer Person, die eine Transplantation benötigt (der Empfänger);
(b) Erhalten von Stammzellen, die für die in (a) identifizierten MHC-Allele homozygot sind, von einer Stammzellbank, die eine Mehrzahl von menschlichen parthenogenetischen Stammzelllinien umfasst, die für mindestens ein MHC-Allel des Transplantatempfängers homozygot sind; und
(c) Erzeugen von zur Transplantation geeigneten Zellen oder Geweben aus den Stammzellen.

36. Verfahren nach Anspruch 35, wobei die Stammzellbank mindestens 10 verschiedene menschliche parthenogenetische Stammzelllinien umfasst, wobei jede der Stammzelllinien für eine unterschiedliche Kombination von HLA-Allelen relativ zu den anderen Stammzelllinien homozygot ist.

37. Verfahren nach Anspruch 35, wobei die Stammzellbank mindestens 15 verschiedene menschliche parthenogenetische Stammzelllinien umfasst, wobei jede der Stammzelllinien für eine unterschiedliche Kombination von HLA-Allelen relativ zu den anderen menschlichen Stammzelllinien in der Zellbank homozygot ist.

38. Verfahren nach Anspruch 35, wobei die Stammzellbank mindestens etwa 100 bis 1.000 menschliche parthenogenetische Stammzelllinien umfasst, die jeweils für eine unterschiedliche Kombination von HLA-Allelen relativ zu den anderen menschlichen Stammzelllinien in der Zellbank homozygot sind.

39. Verfahren nach Anspruch 35, wobei die menschliche Stammzellbank Zellen umfasst, die für einen der folgenden HLA-Serotypen homozygot sind: HLA-A1, HLA-A3, HLA-A11, HLA-A15, HLA-A22, HLA-A27, HLA-A28, HLA-A29, HLA-A32, HLA-B5, HLA-B7, HLA-B8, HLA-B12, HLA-B17, HLA-B18, HLA-B35 und HLA-B40.

40. Verfahren nach Anspruch 35, wobei die menschliche Stammzellbank Stammzellen umfasst, die für mindestens eines der folgenden HLA-A-, -B- oder DR-Haplotypen homozygot sind: 1,7, 2; 1, 8, 3; 2, 14, 1; 2, 35, 4; 2, 35, 8; 2, 44, 4; 3, 7, 2; 3, 7, 4; 3, 7, 8; 3, 35, 1; 31, 51, 4; und 32, 14, 7.

41. Verfahren nach Anspruch 39 oder 40, wobei die Zellbanken O-negativ sind.

42. Verfahren nach Anspruch 35, wobei der Schritt b das Erhalten von Stammzellen umfasst, die sich in hämatopoetische Stammzellen differenzieren können.

43. Verfahren nach Anspruch 35, wobei der Schritt b das Erhalten hämatopoetischer Stammzellen aus der Stammzellbank umfasst.

44. Verfahren nach Anspruch 35, wobei der Schritt b das Erhalten von Stammzellen umfasst, die homozygot für ein MHC-Allel sind, ausgewählt aus HLA-A, HLA-B, HLA-C, HLA-DR, HLA-DQ und HLA-DP.

45. Verfahren nach Anspruch 44, wobei die MHC-Allele HLA-A, HLA-B und HLA-DR kodieren.

46. Verfahren nach Anspruch 35, wobei der Schritt b das Erhalten diploider Stammzellen umfasst, die von durch Parthenogenese hergestellten Embryonen stammen.

47. Verfahren nach Anspruch 46, umfassend das Erhalten diploider Stammzellen, in denen alle MHC-Allele homozygot sind.

48. Verfahren nach Anspruch 35, wobei der Schritt b das Erhalten von Stammzellen mit DNA umfasst, die im Vergleich zu der DNA des menschlichen Spenders, von dem die Stammzellen abgeleitet sind, genetisch modifiziert ist.

49. Verfahren nach Anspruch 48, umfassend das Erhalten genetisch veränderter Stammzellen, deren DNA durch Hinzufügen, Modifizieren, Ersetzen oder Löschen einer oder mehrerer DNA-Sequenzen modifiziert ist.

50. Verfahren nach Anspruch 48, umfassend das Erhalten genetisch veränderter Stammzellen, deren DNA modifiziert ist, um die Expression eines Gens zu erhalten, zu erhöhen, zu verringern, zu hemmen oder auf andere Weise zu modifizieren, das zu den Zellen nativ oder in diese eingeführt ist, relativ zur Expression des Gens in einer Kontrollzelle ohne die genetische Modifikation.

51. Verfahren nach Anspruch 48, umfassend das Erhalten genetisch veränderter Stammzellen, deren DNA durch homologe Rekombination modifiziert ist.

52. Verfahren nach Anspruch 48, umfassend das Erhalten genetisch veränderter Stammzellen, deren DNA verändert wird, um die Expression eines Gens zu verhindern, das ein antigenes Protein kodiert, das eine Immunantwort hervorruft, die zur Abstoßung beiträgt.

53. Verfahren nach Anspruch 51, umfassend genetisch veränderte Stammzellen, deren DNA so modifiziert ist, dass sie die Produktion von mindestens einem HLA-Antigen durch Zellen der Zelllinie hemmt.

54. Verfahren nach Anspruch 53, wobei das mindestens eine HLA-Antigen aus HLA-A, HLA-B, HLA-C, HLA-DR, HLA-DQ und HLA-DP ausgewählt ist.

55. Verfahren nach Anspruch 51, umfassend genetisch veränderte Stammzellen, deren DNA modifiziert ist, um die Produktion von Beta2-Mikroglobulin zu hemmen.

56. Verfahren nach Anspruch 51, umfassend genetisch veränderte Stammzellen, deren DNA durch Ersetzen eines nicht homozygoten MHC-Allels durch ein homozygotes verändert wird.

57. Verfahren zur Produktion von menschlichen Stammzellen, die homozygot für mindestens ein in einer menschlichen Population vorhandenes MHC-Allel sind, umfassend:
(a) Aktivieren von Eizellen;
(b) Aussetzen der aktivierten Eizellen einer chemischen Behandlung, die die Extrusion des zweiten Polkörpers hemmt;
(c) Kultivieren des parthenogenetischen Embryos in vitro unter Bedingungen, die zur Bildung einer Blastozyste führen;
(d) Kultivieren innerer Zellmassenzellen der Blastozyste in vitro unter Bedingungen, die zur Produktion von Stammzellen führen; und
(e) Screening zur Identifizierung von Stammzellen, die in einem bestimmten MHC-Allel homozygot sind.

58. Verfahren nach Anspruch 57, wobei das Verfahren mit Eizellen von verschiedenen Spendern wiederholt wird und eine Bank oder Bibliothek von verschiedenen Stammzelllinien erhalten wird, die für verschiedene MHC-Allele relativ zu den anderen Mitgliedern in der Gruppe homozygot sind.

59. Verfahren nach Anspruch 58, wobei die Stammzellbank Stammzelllinien umfasst, die für einen oder mehrere der folgenden HLA-Serotypen homozygot sind: HLA-A1, HLA-A3, HLA-A11, HLA-A15, HLA-A22, HLA-A24, HLA-A28, HLA-A29, HLA-A32, HLA-B5, HLA-B7, HLA-B8, HLA-B12, HLA-B17, HLA-B18, HLA-B35 und HLA-B40.

60. Verfahren nach Anspruch 58, wobei die Stammzellbank Stammzelllinien umfasst, die für mindestens einen der folgenden HLA-A, -B und -DR-Haplotypen homozygot sind: 1, 7, 2; 1, 8, 3; 2,14, 1; 2, 35, 4; 2, 35, 8; 2, 44, 4; 3, 7, 2; 3, 7, 4; 3, 7, 8; 3, 35, 1; 31, 51, 4 und 32, 14, 7.

61. Verfahren nach Anspruch 58, Anspruch 59 oder Anspruch 60, wobei die Stammzelllinien O-negativ sind.

62. Verfahren nach Anspruch 57, wobei die Stammzellen zu hämatopoetischen Stammzellen differenzieren können.

63. Verfahren nach Anspruch 57, wobei die Stammzellen homozygot für ein MHC-Allel sind, ausgewählt aus HLA-A, HLA-B, HLA-C, HLA-DR, HLA-DQ und HLA-DP.

64. Verfahren nach Anspruch 63, wobei die MHC-Allele für HLA-A, HLA-B und HLA-DR kodieren.

## Revendications

1. Banque de cellules souches comprenant une bibliothèque ou une pluralité de lignées de cellules souches parthénogénétiques humaines, chacune d'elles étant homozygote pour au moins un allèle MHC présent dans une population humaine, chaque élément de ladite pluralité de lignées de cellules souches étant homozygote pour un ensemble différent d'allèles MHC par rapport aux autres éléments de la pluralité de lignées de cellules souches.

2. Banque de cellules souches selon la revendication 1, comprenant au moins cinq lignées de cellules souches différentes, chaque élément étant homozygote pour une combinaison différente d'allèles d'antigènes d'histocompatibilité ou d'allèles d'antigènes MHC.

3. Banque de cellules souches selon la revendication 1, comprenant au moins dix lignées de cellules souches différentes, chaque élément étant homozygote pour une combinaison différente d'allèles d'antigènes d'histocompatibilité ou d'allèles d'antigènes MHC.

4. Banque de cellules souches selon la revendication 1, comprenant au moins environ 100 à 1 000 lignées de cellules souches parthénogénétiques différentes.

5. Banque de cellules souches selon la revendication 1, comprenant des lignées de cellules souches parthénogénétiques humaines qui sont homozygotes au moins pour les 10 allèles MHC les plus fréquents dans la population humaine.

6. Banque de cellules souches selon la revendication 1, comprenant des lignées de cellules souches parthénogénétiques humaines qui sont homozygotes au moins pour les 15 allèles MHC les plus fréquents dans la population humaine.

7. Banque de cellules souches selon la revendication 1, dans laquelle toutes les lignées de cellules souches sont O négatif.

8. Banque de cellules souches selon la revendication 5, dans laquelle la bibliothèque comporte des cellules souches parthénogénétiques humaines qui sont homozygotes au moins pour l'un des allèles MHC suivants : HLA-A1, HLA-A2, HLA-A3, HLA-A24, HLA-A11, HLA-A28, HLA-A29, HLA-A32, HLA-B15, HLA-B5, HLA-B7, HLA-B8, HLA-B12, HLA-B14, HLA-B18, HLA-B35 et HLA-B40.

9. Banque de cellules souches selon la revendication 8, dans laquelle toutes lesdites lignées de cellules souches parthénogénétiques humaines sont O négatif.

10. Banque de cellules selon la revendication 1, comprenant au moins une lignée de cellules souches parthénogénétiques humaines rendue homozygote pour un allèle MHC par élimination d'un ou de plusieurs allèles par knock-out ou par interférence ARN.

11. Banque de cellules selon la revendication 5, comprenant des lignées de cellules souches qui sont homozygotes au moins pour l'une des combinaisons suivantes d'haplotypes HLA-A, HLA-B et HLA-DR : 1, 7, 2 ; 1, 8, 3 ; 2, 14, 1 ; 2, 35, 4 ; 2, 35, 8 ; 2, 44, 4 ; 3, 7, 2 ; 3, 7, 4 ; 3, 7, 8 ; 3, 35, 1 ; 31, 51, 4 ; et 32, 14, 7.

12. Banque de cellules souches selon la revendication 11, contenant des lignées de cellules homozygotes pour toutes lesdites combinaisons d'haplotypes HLA-A, HLA-B et HLA-DR.

13. Banque de cellules souches selon la revendication 11 ou 12, laquelle banque est O négatif.

14. Banque de cellules souches selon la revendication 10, dans laquelle les cellules sont rendues homozygotes par élimination d'un ensemble d'allèles MHC par knock-out ou par interférence ARN, et par ajout d'un autre ensemble homozygote d'allèles MHC.

15. Banque de cellules souches selon la revendication 1, contenant une pluralité de lignées de cellules souches capables de se transformer en cellules souches hématopoïétiques.

16. Banque de cellules souches selon la revendication 1, contenant une pluralité de lignées de cellules souches hématopoïétiques.

17. Banque de cellules souches selon la revendication 1, contenant une pluralité de lignées de cellules souches capables de se transformer en cellules précurseurs endothéliales vasculaires.

18. Banque de cellules souches selon la revendication 1 contenant une pluralité de cellules souches capables de se transformer en cellules myocardiques.

19. Banque de cellules souches selon la revendication 1 contenant une pluralité de cellules souches capables de se transformer en cellules hépatiques.

20. Banque de cellules souches selon la revendication 1, contenant une pluralité de cellules souches capables de se transformer en cellules bêta pancréatiques.

21. Banque de cellules souches selon la revendication 1, contenant une lignée de cellules souches homozygote pour un allèle MHC choisi parmi HLA-A, HLA-B, HLA-C, HLA-DR, HLA-DQ et HLA-DP, les cellules étant du groupe sanguin O négatif.

22. Banque de cellules souches selon la revendication 1, contenant une lignée de cellules souches homozygote pour les allèles MHC codant pour HLA-A, HLA-B et HLA-DR.

23. Banque de cellules souches selon la revendication 22, dans laquelle la lignée de cellules souches est du groupe sanguin O négatif.

24. Banque de cellules souches selon la revendication 1, contenant des lignées de cellules souches diploïdes dont tous les allèles MHC sont homozygotes.

25. Banque de cellules souches selon la revendication 1, contenant au moins une lignée de cellules souches parthénogénétiques humaines qui comprend de l'ADN génétiquement modifié par rapport à celui du donneur humain de qui la lignée de cellules est dérivée.

26. Banque de cellules souches selon la revendication 25, dans laquelle l'ADN ou la lignée de cellules souches génétiquement modifié(e) est modifié(e) par ajout, modification, substitution ou suppression d'une ou de plusieurs séquences d'ADN.

27. Banque de cellules souches selon la revendication 25, dans laquelle l'ADN de la lignée de cellules souches génétiquement modifiée est modifié de manière à obtenir, à augmenter, à diminuer, à inhiber ou autrement à modifier l'expression d'un gène endogène à, ou introduit dans des cellules de l'au moins une lignée de cellules, par rapport à l'expression dudit gène dans une cellule témoin sans modification génétique.

28. Banque de cellules souches selon la revendication 25, dans laquelle l'ADN de la lignée de cellules souches génétiquement modifiée est modifié par recombinaison homozygote.

29. Banque de cellules souches selon la revendication 25, dans laquelle l'ADN de la lignée de cellules souches génétiquement modifiée est modifié de façon à empêcher l'expression d'un gène codant pour une protéine antigénique qui déclenche une réponse immunitaire contribuant au rejet.

30. Banque de cellules souches selon la revendication 25, dans laquelle l'ADN de la lignée de cellules souches génétiquement modifiée est modifié de façon à exprimer un gène qui inhibe le rejet immunitaire de cette cellule.

31. Banque de cellules souches selon la revendication 28, dans laquelle l'ADN de la lignée de cellules souches génétiquement modifiée est modifié de façon à inhiber la production d'au moins un antigène HLA par les cellules de ladite lignée de cellules.

32. Banque de cellules souches selon la revendication 31, dans laquelle l'au moins un antigène est choisi parmi HLA-A, HLA-B, HLA-C, HLA-DR, HLA-DQ et HLA-DP.

33. Banque de cellules souches selon la revendication 28, dans laquelle l'ADN de la lignée de cellules souches génétiquement modifiée est modifié de manière à inhiber la production de la bêta-2-microglobuline.

34. Banque de cellules souches selon la revendication 28, dans laquelle l'ADN de la lignée de cellules souches génétiquement modifiée est modifié par remplacement d'un allèle MHC non homozygote par un allèle MHC homozygote.

35. Procédé in vitro de préparation d'une cellule ou d'un tissu pour une transplantation, consistant à préparer des cellules ou des tissus pour une transplantation, lesquels sont homozygotes pour au moins un allèle HLA d'une personne qui a besoin d'une telle transplantation, consistant à :
a) identifier les allèles MHC d'une personne ayant besoin d'une transplantation (le receveur) ;
b) prélever des cellules souches qui sont homozygotes pour les allèles MHC identifiés à l'étape a), à partir de la banque de cellules souches comprenant une pluralité de lignées de cellules souches parthénogénétiques humaines homozygotes pour au moins un allèle MHC du receveur de la transplantation ; et à
c) créer, à partir desdites cellules souches, des cellules ou des tissus adaptés pour une transplantation.

36. Procédé selon la revendication 35, dans lequel ladite banque de cellules souches comprend au moins 10 lignées de cellules souches parthénogénétiques humaines différentes, chacune desdites lignées de cellules souches étant homozygote pour une combinaison différente d'allèles HLA par rapport aux autres lignées de cellules souches.

37. Procédé selon la revendication 35, dans lequel ladite banque de cellules souches comprend au moins 15 lignées de cellules souches parthénogénétiques humaines différentes, chacune desdites lignées de cellules souches étant homozygote pour une combinaison différente d'allèles HLA par rapport aux autres lignées de cellules souches humaines de la banque de cellules.

38. Procédé selon la revendication 35, dans lequel ladite banque de cellules souches comprend au moins environ 100 à 1 000 lignées de cellules souches parthénogénétiques humaines, chacune étant homozygote pour une combinaison différente d'allèles HLA par rapport aux autres lignées de cellules souches humaines de la banque de cellules.

39. Procédé selon la revendication 35, dans lequel ladite banque de cellules souches humaines comprend des cellules qui sont homozygotes pour l'un des sérotypes HLA suivants : HLA-A1, HLA-A3, HLA-A11, HLA-A15, HLA-A22, HLA-A27, HLA-A28, HLA-A29, HLA-A32, HLA-B5, HLA-B7, HLA-B8, HLA-B12, HLA-B17, HLA-B18, HLA-B35 et HLA-B40.

40. Procédé selon la revendication 35, dans lequel ladite banque de cellules souches humaines comprend des cellules souches qui sont homozygotes au moins pour l'un des haplotypes HLA-A, HLA-B ou HLA-DR suivants : 1, 7, 2 ; 1, 8, 3 ; 2, 14, 1 ; 2, 35, 4 ; 2, 35, 8 ; 2, 44, 4 ; 3, 7, 2 ; 3, 7, 4 ; 3, 7, 8 ; 3, 35, 1 ; 31, 51, 4 ; et 32, 14, 7.

41. Procédé selon la revendication 39 ou 40, dans lequel lesdites banques de cellules sont O négatif.

42. Procédé selon la revendication 35, dans lequel l'étape b consiste à prélever des cellules souches capables de se transformer en des cellules souches hématopoïétiques.

43. Procédé selon la revendication 35, dans lequel l'étape b consiste à prélever des cellules souches hématopoïétiques à partir de la banque de cellules souches.

44. Procédé selon la revendication 35, dans lequel l'étape b consiste à prélever des cellules souches homozygotes pour un allèle MHC choisi parmi HLA-A, HLA-B, HLA-C, HLA-DR, HLA-DQ et HLA-DP.

45. Procédé selon la revendication 44, dans lequel les allèles MHC codent pour HLA-A, HLA-B et HLA-DR.

46. Procédé selon la revendication 35, dans lequel l'étape b consiste à prélever des cellules souches diploïdes dérivées d'embryons produits par parthénogenèse.

47. Procédé selon la revendication 46, consistant à prélever des cellules souches diploïdes dont tous les allèles MHC sont homozygotes.

48. Procédé selon la revendication 35, dans lequel l'étape b consiste à prélever des cellules souches présentant un ADN génétiquement modifié par rapport à l'ADN du donneur humain de qui les cellules souches sont dérivées.

49. Procédé selon la revendication 48, consistant à prélever des cellules souches génétiquement modifiées dont l'ADN est modifié par ajout, modification, substitution ou suppression d'une ou de plusieurs séquences d'ADN.

50. Procédé selon la revendication 48, consistant à prélever des cellules souches génétiquement modifiées dont l'ADN est modifié de manière à obtenir, à augmenter, à diminuer, à inhiber ou autrement à modifier l'expression d'un gène endogène à, ou introduit dans lesdites cellules, par rapport à l'expression dudit gène dans une cellule témoin sans modification génétique.

51. Procédé selon la revendication 48, consistant à prélever des cellules souches génétiquement modifiées dont l'ADN est modifié par recombinaison homologue.

52. Procédé selon la revendication 48, consistant à prélever des cellules souches génétiquement modifiées dont l'ADN est modifié de façon à empêcher l'expression d'un gène codant pour une protéine antigénique qui déclenche une réponse immunitaire contribuant au rejet.

53. Procédé selon la revendication 51, comprenant des cellules souches génétiquement modifiées dont l'ADN est modifié de façon à inhiber la production d'au moins un antigène HLA par des cellules de ladite lignée de cellules.

54. Procédé selon la revendication 53, dans laquelle l'au moins un antigène est choisi parmi HLA-A, HLA-B, HLA-C, HLA-DR, HLA-DQ et HLA-DP.

55. Procédé selon la revendication 51, comprenant des cellules souches génétiquement modifiées dont l'ADN est modifié de façon à inhiber la production de la bêta-2-microglobuline.

56. Procédé selon la revendication 51, comprenant des cellules souches génétiquement modifiées dont l'ADN est modifié par remplacement d'un allèle MHC non homozygote par un allèle MHC homozygote.

57. Procédé de production de cellules souches humaines homozygotes au moins pour un allèle MHC présent dans une population humaine, consistant à :
a) activer des ovocytes ;
b) exposer les ovocytes activés à un traitement chimique qui inhibe l'extrusion du second globule polaire ;
c) cultiver in vitro l'embryon parthénogénétique dans des conditions entraînant la formation d'un blastocyste ;
d) cultiver in vitro des cellules de masse cellulaire interne du blastocyste dans des conditions entraînant la production de cellules souches ; et à
e) procéder à un criblage afin d'identifier des cellules souches homozygotes dans un allèle MHC particulier.

58. Procédé selon la revendication 57, ledit procédé étant répété avec des ovocytes provenant de différents donneurs, et dans lequel une banque ou bibliothèque de lignées de cellules souches différentes est obtenue, lesdites lignées étant homozygotes pour différents allèles MHC par rapport aux autres éléments du groupe.

59. Procédé selon la revendication 58, dans lequel ladite banque de cellules souches comprend des lignées de cellules souches qui sont homozygotes pour un ou plusieurs des sérotypes HLA suivants : HLA-A1, HLA-A3, HLA-A11, HLA-A15, HLA-A22, HLA-A24, HLA-A28, HLA-A29, HLA-A32, HLA-B5, HLA-B7, HLA-B8, HLA-B12, HLA-B17, HLA-B18, HLA-B35 et HLA-B40.

60. Procédé selon la revendication 58, dans lequel ladite banque de cellules souches comprend des lignées de cellules souches qui sont homozygotes au moins pour l'un des haplotypes HLA-A, HLA-B ou HLA-DR suivants : 1, 7, 2 ; 1, 8, 3 ; 2, 14, 1 ; 2, 35, 4 ; 2, 35, 8 ; 2, 44, 4; 3, 7, 2 ; 3, 7, 4; 3, 7, 8 ; 3, 35, 1 ; 31, 51, 4 et 32, 14, 7.

61. Procédé selon la revendication 58, 59 ou 60, dans lequel lesdites lignées de cellules souches sont O négatif.

62. Procédé selon la revendication 57, dans lequel les cellules souches sont capables de se transformer en cellules souches hématopoïétiques.

63. Procédé selon la revendication 57, dans lequel les cellules souches sont homozygotes pour un allèle MHC choisi parmi HLA-A, HLA-B, HLA-C, HLA-DR, HLA-DQ et HLA-DP.

64. Procédé selon la revendication 63, dans lequel les allèles MHC codent pour HLA-A, HLA-B et HLA-DR.
